(19) Europäisches Patentamt
European Patent Office
Office européen des brevets

(11) EP 0 880 962 B1

(12) FASCICULE DE BREVET EUROPEEN

(45) Date de publication et mention de la délivrance du brevet:
**31.01.2001 Bulletin 2001/05**

(51) Int Cl.[7]: **A61K 7/42**, A61K 7/48

(21) Numéro de dépôt: **98400993.6**

(22) Date de dépôt: **23.04.1998**

# (54) Composition comprenant un dérivé de dibenzoylméthane et un polymère polyamine

Ein Dibenzoylmethanderivat und ein Polyamin-Polymer enthaltende Zusammensetzungen

Compositions comprising a derivative of dibenzoylmethane and a polymino-polymer

(84) Etats contractants désignés:
**DE ES FR GB IT**

(30) Priorité: **28.05.1997 FR 9706531**

(43) Date de publication de la demande:
**02.12.1998 Bulletin 1998/49**

(73) Titulaire: **L'OREAL**
**75008 Paris (FR)**

(72) Inventeurs:
- **Boussouira, Boudiaf**
  **75020 Paris (FR)**
- **Candau, Didier**
  **91570 Bievres (FR)**

(74) Mandataire: **Miszputen, Laurent**
**L'OREAL-DPI**
**6 rue Bertrand Sincholle**
**92585 Clichy Cédex (FR)**

(56) Documents cités:
**EP-A- 0 782 846** **WO-A-93/04666**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouvelles compositions cosmétiques et/ou dermatologiques (ci-après appelées compositions antisolaires) destinées à la protection de la peau et/ou des cheveux contre les rayonnements UV, en particulier le rayonnement solaire. Plus précisément, elle concerne de nouvelles compositions cosmétiques et/ou dermatologiques présentant une photostabilité améliorée et comprenant, dans un support cosmétiquement et/ou dermatologiquement acceptable, l'association d'au moins un filtre UV dérivé de dibenzoylméthane et au moins un polymère polyaminé.

L'invention concerne également l'utilisation de ces compositions dans les domaines cosmétique et/ou dermatologique.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UVA et de filtres actifs dans l'UVB.

A cet égard, une famille de filtres UV-A particulièrement intéressante est actuellement constituée par les dérivés du dibenzoylméthane, et notamment le 4-ter-butyl-4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A- 2 326 405 et FR-A- 2 440 933, ainsi que dans la demande de brevet européen EP-A- 0 114 607.

Toutefois, la Demanderesse a constaté que ces dérivés du dibenzoylméthane, en particulier du 4-tert-butyl-4'-méthoxydibenzoylméthane, lorsqu'ils sont exposés à des irradiations UV, présentent l'inconvénient de se dégrader chimiquement de façon importante. En particulier, la demanderesse a constaté que ces dérivés du dibenzoylméthane subissaient des réactions de peroxydation lorsqu'ils étaient exposés aux UV.

Outre le problème de la photo-dégradation des dérivés de dibenzoylméthane eux-mêmes, la demanderesse a constaté que le dibenzoylméthane, lorsqu'il est associé à certains autres filtres organiques, présente l'inconvénient de provoquer la photo-dégradation de ces filtres lorsque cette association est exposée à des rayonnements UV.

Les dérivés de 1,3,5-triazine, et en particulier la 2,4,6-tris[p-(2'-éthylhexyl-1'-oxycarbonyl)anilino]-1,3,5-triazine, vendue sous la dénomination commerciale « UVINUL T 150 » par la société BASF possèdent un fort pouvoir absorbant des UVB. Il serait donc très intéressant de pouvoir les utiliser en association avec le 4-tert-butyl-4'-méthoxydibenzoylméthane cité ci-dessus dans le but d'obtenir des produits offrant une protection large et efficace dans l'ensemble du rayonnement UV.

Toutefois, la Demanderesse a constaté que ces dérivés de 1,3,5-triazine, lorsqu'ils sont mis en présence de dérivés du dibenzoylméthane, en particulier du 4-tert-butyl-4'-méthoxydibenzoylméthane, et placés sous irradiation UV, présentent l'inconvénient de se dégrader chimiquement de façon importante. Dans ces conditions, l'association des deux filtres ne permet plus une protection solaire large prolongée de la peau et des cheveux.

Or, à la suite d'importantes recherches menées dans le domaine de la photoprotection évoqué ci-dessus, la Demanderesse a maintenant découvert que :

- l'association de certains polymères polyaminés, qui seront définis ci-dessous, avec un dérivé de dibenzoylméthane, en particulier du 4-tert-butyl-4'-méthoxydibenzoylméthane, permettait d'améliorer de façon tout à fait remarquable la photostabilité de ce dérivé de dibenzoylméthane lorsqu'il est exposé à un rayonnement UV et donc d'améliorer l'efficacité globale de ces filtres ;

- l'association de certains polymères polyaminés, qui seront définis ci-dessous, avec un dérivé de dibenzoylméthane et un autre filtre organique choisi parmi les dérivés de 1,3,5-triazine permettait d'améliorer de façon tout à fait

remarquable la photostabilité des dérivés de 1,3,5-triazine en présence de ce dérivé de dibenzoylméthane lorsque l'association est exposée à un rayonnement UV et donc d'améliorer l'efficacité globale de l'association filtrante dérivé de dibenzoylméthane + dérivé de 1,3,5-triazine.

La présente invention a donc pour objet de nouvelles compositions cosmétiques et/ou dermatologiques comprenant, dans un support cosmétiquement et/ou dermatologiquement acceptable :

a) au moins un polymère polyaminé tel que défini ci-après ;
b) au moins un dérivé de dibenzoylméthane répondant à la formule (V) suivante :

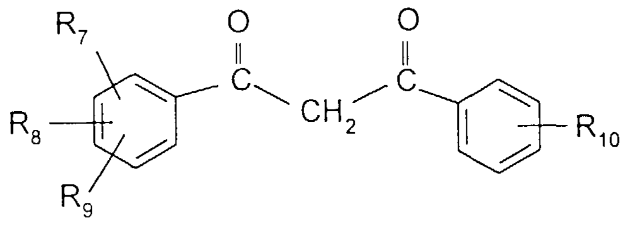

(V)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$.

Ainsi, selon la présente invention, on peut réaliser des compositions cosmétiques et/ou dermatologiques contenant un dérivé de dibenzoylméthane, en particulier du 4-tert-butyl-4'-méthoxydibenzoylméthane, compositions dans lesquelles la concentration en dérivé de dibenzoylméthane reste relativement constante même si ces compositions sont soumises à l'action de la lumière.

L'invention a également pour objet une composition cosmétique et/ou dermatologique comprenant, dans un support cosmétiquement et/ou dermatologiquement acceptable :

a) au moins un polymère polyaminé ;
b) au moins un dérivé de dibenzoylméthane répondant à la formule (V) suivante :

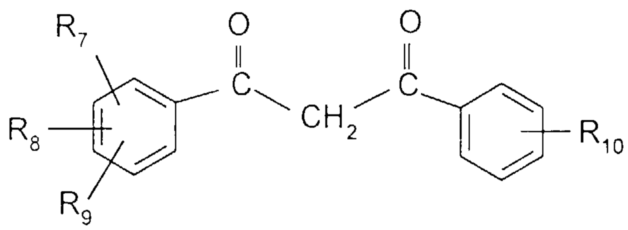

(V)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$ ;
c) au moins un dérivé de 1,3,5-triazine répondant à la formule (I) suivante :

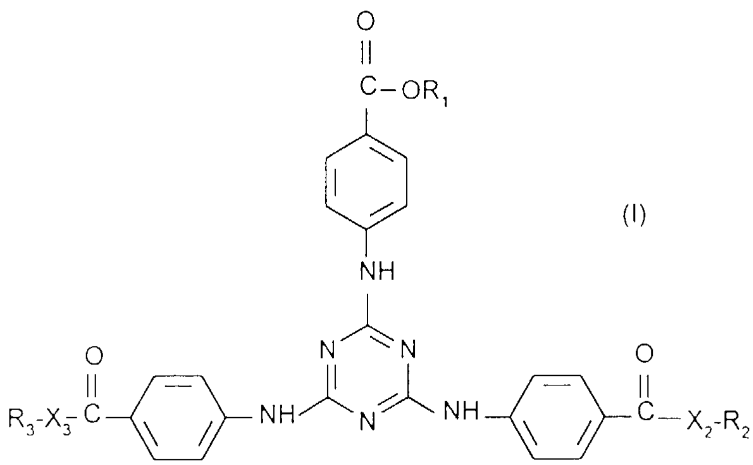

(I)

dans laquelle :

- $X_2$ et $X_3$, identiques ou différents, représentent l'oxygène ou le radical -NH- ;
- $R_1$, $R_2$ et $R_3$, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en $C_1$-$C_{18}$; un radical cycloalkyle en $C_5$-$C_{12}$ éventuellement substitué par un ou plusieurs radicaux alkyles en $C_1$-$C_4$; un radical polyoxyéthyléné comprenant de 1 à 6 unités d'oxyde d'éthylène et dont le groupe OH terminal est méthylé; un radical de formule (II), (III) ou (IV) suivantes :

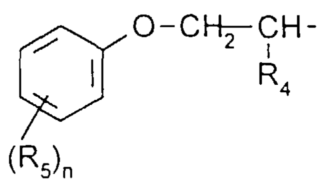

(II)

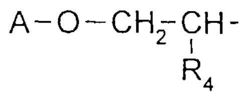

(III)

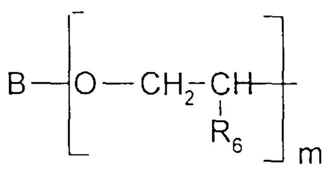

(IV)

dans lesquelles :

- $R_4$ est l'hydrogène ou un radical méthyle ;
- $R_5$ est un radical alkyle en $C_1$-$C_9$ ;
- n est un nombre entier allant de 0 à 3 ;
- m est un nombre entier allant de 1 à 10 ;
- A est un radical alkyle en $C_4$-$C_8$ ou un radical cycloalkyle en $C_5$-$C_8$ ;
- B est choisi parmi : un radical alkyle linéaire ou ramifié en $C_1$-$C_8$ ; un radical cycloalkyle en $C_5$-$C_8$ ; un radical aryle éventuellement substitué par un ou plusieurs radicaux alkyles en $C_1$-$C_4$ ;

- $R_6$ est l'hydrogène ou un radical méthyle.

Bien entendu, dans la définition ci-dessus, lorsque $X_2$ et/ou $X_3$ représentent un radical -NH-, alors le ou les radicaux $R_2$ et/ou $R_3$ correspondants sont différents d'un métal alcalin ou d'un radical ammonium.
Ainsi, selon la présente invention, on peut réaliser des compositions cosmétiques et/ou dermatologiques contenant un dérivé de dibenzoylméthane, en particulier du 4-tert-butyl-4'-méthoxydibenzoylméthane, en association avec au moins un dérivé de 1,3,5-triazine, compositions dans lesquelles la concentration en dérivé de 1,3,5-triazine reste relativement constante même si ces compositions sont soumises à l'action de la lumière.
La présente invention a encore pour objet l'utilisation d'un polymère polyaminé dans, ou pour la fabrication de, compositions cosmétiques et/ou dermatologiques contenant un dérivé de dibenzoylméthane tel que défini ci-dessus en vue d'améliorer dans lesdites compositions la stabilité au rayonnement UV (photostabilité) dudit dérivé de dibenzoylméthane.
La présente invention a également pour objet un procédé pour améliorer la stabilité au rayonnement UV (photostabilité), et donc l'efficacité, d'une composition cosmétique et/ou dermatologique comprenant un dérivé de dibenzoylméthane tel que défini ci-dessus, ledit procédé consistant à introduire dans ladite composition une quantité efficace d'un polymère polyaminé.
La présente invention a encore pour objet l'utilisation d'un polymère polyaminé tel que défini ci-dessus dans, ou pour la fabrication de, compositions cosmétiques et/ou dermatologiques contenant un dérivé de dibenzoylméthane, en association avec au moins un dérivé de 1,3,5-triazine tels que définis ci-dessus en vue d'améliorer dans lesdites compositions la stabilité au rayonnement UV (photostabilité) dudit dérivé de 1,3,5-triazine.
La présente invention a également pour objet un procédé pour améliorer la stabilité au rayonnement UV (photostabilité), et donc l'efficacité, d'une composition cosmétique et/ou dermatologique comprenant un dérivé de dibenzoylméthane et un dérivé de 1,3,5-triazine tels que définis ci-dessus, ledit procédé consistant à introduire dans ladite composition une quantité efficace d'un polymère polyaminé tel que défini ci-dessus.
Par quantité efficace de polymère polyaminé, on entend une quantité suffisante pour obtenir une amélioration notable et significative de la photostabilité du ou des dérivés du dibenzoylméthane contenus dans la composition. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.
D'autres caractéristiques, aspects et avantages de la présente invention apparaîtront à la lecture de la description détaillée qui va suivre.
Les polymères polyaminés de l'invention sont choisis parmi les familles suivantes :

(A) une polyalkylène polyamine ou un dérivé de polyalkylène polyamine choisi parmi :

(i) les polyalkylène polyamines ;
(ii) les dérivés alkylés de polyalkylène polyamines (A)(i) ;
(iii) les produits d'addition d'acides alkylcarboxyliques sur les polyalkylènepolyamines (A)(i) ;
(iv) les produits d'addition de cétones et d'aldéhydes sur les polyalkylènepolyamines (A)(i) ;
(v) les produits d'addition d'isocyanates et d'isothiocyanates sur les polyalkylènepolyamines (A)(i) ;
(vi) les produits d'addition d'oxyde d'alkylène ou de polymères blocs polyoxyde d'alkylène sur les polyalkylènepolyamines (A)(i) ;
(vii) les dérivés quaternisés de polyalkylène polyamines (A)(i) ;
(viii) les produits d'addition d'une silicone sur les polyalkylènepolyamines (A)(i) ;
(ix) un copolymère d'acide dicarboxylique et de polyalkylène polyamines (A)(i) ;

(B) les polyvinylimidazoles ;
(C) les polyvinylpyridines ;
(D) les produits d'addition de monomères 1-vinylimidazole de formule (1):

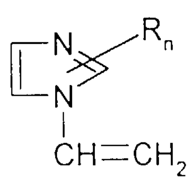
(1)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(ix) ;
(E) les polymères à base d'acides aminés à chaîne latérale basique ;
(F) les dérivés réticulés des polymères (A)(i) à (A)(ix), (B) (C) (D) et (E).

Les polymères polyaminés utilisables dans la présente invention peuvent se trouver sous la forme de polymère linéaire, de polymère hyperbranché ou de dendrimère.

Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications ; une couche de chaînes terminales.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%. On peut faire réagir un groupement terminal T sur le polymère hyperbranché pour obtenir une fonctionnalité particulière en extrémité de chaînes.

De tels polymères sont décrits en particulier dans B.I.Voit, Acta Polymer., 46, 87-99 (1995) ; EP-682059 ; WO-9614346 ; WO-9614345 ; WO-9612754.

Plusieurs polymères hyperbranchés peuvent être associés entre eux, par une liaison covalente ou un autre type de liaison, par l'intermédiaire de leurs groupes terminaux. De tels polymères dits pontés ou « bridged » entrent dans la définition des polymères hyperbranchés selon la présente invention.

Les dendrimères sont des polymères et oligomères hautement ramifiés, également connus, ayant une structure chimique bien définie, et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles , qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la $N^{ième}$ génération sont les groupes fonctionnels terminaux des fuseaux de la Nième génération ou génération terminale. De tels polymères sont décrits en particulier dans D.A.Tomalia, A.M.Naylor et W.A. Goddard III, *Angewandte Chemie,* Int.Ed.Engl.29, 138-175 (1990) ; C.J.Hawker et J.M.J.Frechet, *J.Am.Chem.Soc.,* 112, 7638 (1990) ; B.I.Voit, Acta Polymer., 46, 87-99 (1995) ; N.Ardoin et D.Astruc, Bull. Soc. Chim. Fr. 132, 875-909 (1995).

On peut également définir plus particulièrement les dendrimères par la formule (DI) suivante :

$$C[A_1B_1(A_2B_2(...(A_{n-1}\ B_{n-1}(A_nB_n(T)r_n)r_{n-1})r_{n-2}...)r_2)r_1]s \qquad (DI)$$

dans laquelle :

- C représente le coeur, relié par un nombre s de fonctionnalités à s fuseaux $A_1B_1$ par l'intermédiaire des groupements $A_1$ ;
- s est un nombre entier supérieur ou égal à 1 et inférieur ou égal au nombre de fonctionnalités de C ;
- l'indice i (i=1, 2....n) est un nombre entier qui désigne la génération de chaque fuseau ;
- $r_i$ (i=1, 2.....n-1) représente le nombre de fonctionnalités du groupement $B_i$ appartenant au fuseau ($A_iB_i$), $r_i$ étant un entier supérieur ou égal à 2
- pour chaque fuseau ($A_iB_i$) (i=1, 2.....n), le groupement $B_i$ est relié à $r_i$ groupements $A_{i+1}$ d'un fuseau ($A_{i+1}B_{i+1}$) ;
- chaque groupement $A_i$ (i ≥ 2) est relié à un seul groupement $B_{i-1}$ du fuseau ($A_{i-1}B_{i-1}$) ;
- le fuseau de $n^{ième}$ génération $A_nB_n$ est chimiquement lié à un nombre $r_n$ de groupes terminaux T, $r_n$ étant un entier supérieur ou égal à zéro.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines $\alpha$ et $\varepsilon$ de la lysine, ce qui conduit à une différence dans la longueur des fuseaux des différentes ramifications.

Les polymères denses en étoiles, ou « dense star polymer », les polymères éclatés en étoile, ou «starburst polymer», les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou «bridged dendrimer». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

On peut se reporter aux documents suivants dans lesquels sont décrits des dendrimères comportant des groupements fonctionnels amines : US-4,694,064 ; US-4,631,337; WO-A-9502008 ; WO-A-9314147 ; US-4,360,646 ; Proc. Natl. Acad. Sci. USA, 85, 5409-5413 (1988).

Les polymères hyperbranchés et les dendrimères à groupes fonctionnels aminés peuvent également être constitués d'un coeur et de générations d'unités de base, monomères ou fuseaux, de toutes natures, sur lesquels un groupe terminal T porteur d'une fonction amine a été greffé.

On va décrire de façon plus approfondie les polymères polyaminés (A)(i) à (A)(ix), (B), (C), (D), (E) et (F) de l'invention :

(A) (i)les polyalkylène polyamines utilisés préférentiellement selon l'invention sont des polymères contenant de 7 à 20000 motifs de répétition. De préférence, on choisit des polyalkylène polyamines comprenant au moins 5% d'amines tertiaires, avantageusement au moins 10% de fonctions amines tertiaires, et encore plus préférentiellement au moins 20%. Ces polymères peuvent être des homopolymères ou des copolymères, linéaires, branchés ou de structures dendrimériques.

Ces polymères comprennent les motifs répétitifs suivants :

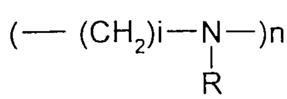

dans lequel :

i représente un entier supérieur ou égal à 2, préférentiellement i=2 ;
n représente un entier
R représente H ou un motif $-(CH_2)_j-$Nè dans lequel j représente un entier supérieur ou égal à 2, préférentiellement j=2 ; Parmi les produits de la famille des polyalkylène polyamines, appelés aussi polyaziridines, on peut citer en particulier:
La polyéthylèneimine, qui est un polymère hyperbranché bien connu de l'homme du métier: au sujet de la polyéthylèneimine on peut se reporter en particulier aux documents : « KIRK-OTHMER ENCYCLOPEDIA OF CHEMICAL TECHNOLOGY », 3ème édition, vol.20, 1982, p214-216 et "Polyéthylèneimine Prospective Application" H.N. Feigenbaum, Cosmetic & Toiletries, 108, 1993, p 73. La polyéthylèneimine est disponible commercialement auprès de la société BASF sous les noms de marque LUPASOL et POLYIMIN; la polyéthylèneimine est habituellement comprise dans une gamme de poids moléculaire moyen allant de 500 à 2.000.000 ;

On connaît aussi des polyéthylèneimines et des polypropylèneimines sous la forme de dendrimères, fabriqués par la société DSM. Les demandes de brevet WO 95/02008 et WO 93/14147 décrivent des polyalkylènepolyamines de la famille des dendrimères ainsi qu'un procédé pour leur préparation.

(A) (ii) les dérivés alkylés de polyalkylène polyamine sont des produits bien connus de l'homme du métier. Ils sont obtenus de façon connue par alkylation, en milieu aqueux ou alcoolique, en présence d'un agent alkylant, de préférence en présence de NaOH, de KOH ou de carbonate, à des températures allant de préférence de 40°C à

130°C. L'agent alkylant peut être choisi par exemple parmi des dérivés sulfate d'alkyle ou halogénure d'alkyle en $C_1$-$C_8$, comme par exemple le diméthylsulfate, le diéthylsulfate, le bromure de butyle, le bromure d'hexyle, le bromure de 2-éthylhexyle, le bromure de n-octyle ou les chlorures correspondants. On peut par exemple se reporter à DE-3743744 qui décrit la préparation de tels produits.

(A) (iii) Les produits d'addition d'acides alkylcarboxyliques sur des polyalkylène polyamines sont des produits connus de l'homme du métier et dont la préparation est décrite par exemple dans les demandes de brevet WO 94/14873 ; WO 94/20681 ; WO 94/12560. L'addition d'acides alkylcarboxyliques sur des polyalkylènepolyamines peut être effectuée en faisant réagir de façon connue un acide, un amide, un ester, un halogénure d'acide sur le polymère polyalkylènepolyamine.

Les produits d'addition d'acides alkylcarboxyliques sur des polyalkylènepolyamines, peuvent être par exemple les produits d'addition d'acides alkylcarboxyliques en $C_2$-$C_{30}$, saturés ou insaturés, linéaires ou ramifiés, sur une polyéthylèneimine. Parmi les acides carboxyliques utilisables, on peut citer par exemple, l'acide acétique, l'acide propionique, l'acide butyrique, l'acide 2-éthylhexanoïque, l'acide benzoïque, l'acide laurique, l'acide myristique, l'acide palmitique, l'acide stéarique, l'acide oléique, l'acide linoléique, l'acide arachidonique, l'acide béhénique, ainsi que les mélanges de corps gras, comme par exemple les mélanges d'esters gras disponibles sous forme de produits naturels, et parmi lesquels on peut citer : l'huile de coco, l'huile de soja, l'huile de lin, l'huile de colza.

(A) (iv) les produits d'addition de cétones et d'aldéhydes sur les polyalkylènepolyamines (A)(i) peuvent être préparés par des procédés connus de l'homme du métier et conduisent à l'obtention de motifs $\alpha$-hydroxyamine ;

(A) (v) les produits d'addition d'isocyanates et d'isothiocyanates sur les polyalkylènepolyamines (A)(i) peuvent être préparés par des procédés connus de l'homme du métier et conduisent à l'obtention de motifs urée et thio-urée ;

(A) (vi) les produits d'addition d'oxyde d'alkylène ou de polymères blocs polyoxyde d'alkylène sur les polyalkylènepolyamines (A)(i) peuvent être préparés par des procédés connus de l'homme du métier ; on peut se reporter par exemple aux documents EP-541018 et US-4,144,123, dans lesquels sont décrites de telles molécules ; des dérivés de polyéthylèneimine éthoxylés sont disponibles commercialement sous le nom de marque : LUPASOL 61(BASF)

(A) (vii) les dérivés quaternisés de polyalkylène polyamines (A)(i) peuvent être préparés par des procédés connus de l'homme du métier;

(A) (viii) les produits d'addition d'une silicone sur les polyalkylènepolyamines (A)(i) sont par exemple des polyéthylèneimines greffées par des motifs polydiméthylsiloxane dont la préparation est décrite dans le document US-5,556,616 et commercialisées par la société MAC INTYRE sous le nom de marque MACKAMER PAVS ;

(A) (ix) les copolymères d'acide dicarboxylique et de polyalkylène polyamines (A)(i) peuvent être préparés par polycondensation d'acides dicarboxyliques avec des polyalkylène polyamines.

Parmi les acides dicarboxyliques pouvant être utilisés pour la préparation des polyamidoamines on peut citer les acides dicarboxyliques en $C_2$ à $C_{10}$, comme par exemple l'acide oxalique, l'acide malonique, l'acide itaconique, l'acide succinique, l'acide maléique, l'acide adipique, l'acide glutarique, l'acide sébacique, l'acide téréphtalique, l'acide orthophtalique, ainsi que leurs mélanges.

Les polyalkylène polyamines utilisées pour la préparation des polyamidoamines sont avantageusement choisies parmi celles ayant de 3 à 10 atomes d'azote, comme par exemple la diéthylène tri-amine, la triéthylène tétramine, la dipropylène tri-amine, la tripropylène tétramine, la di-hexaméthylène triamine, l'amino propyléthylène di-amine, la bis-aminopropyléthylène di-amine ainsi que leurs mélanges. On peut également utiliser des polyéthylène imines telles que décrites ci-dessus pour la préparation de polyamidoamines.

De tels composés sont décrits par exemple dans les documents : US 4,144,423 et WO 94/29422.

(B) le terme polyvinylimidazole comprend les homopolymères et les copolymères de polyvinylimidazole (PVI) obtenus par polymérisation radicalaire des monomères de vinylimidazole de structure suivante :

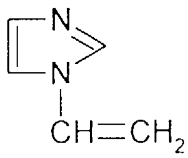

Les copolymères peuvent être par exemple des copolymères de vinylimidazole comportant au moins 5% de motifs vinylimidazole avec des monomères choisis parmi les motifs : vinylpyrrolidinone, acide acrylique et acrylamide. La synthèse de tels composés est bien connue de l'homme du métier; à ce sujet, on peut en particulier se reporter aux documents : J.Am.Chem.Soc., vol.85, 1962, p.951 ; Polymer Letters Ed., vol.11, 1973, p.465-469 ; Macromolecules, vol.6(2), 1973, p.163-168; Ann.N.Y.Acad.Sci., vol.155, 1969, p.431; FR-A-1,477,147 ; JP-69 07395 ; J. Macromol.Scien.Chem., vol.A21(2), 1984, p.253.

(C) le terme polyvinylpyridine comprend les homopolymères et les copolymères de vinyl pyridine obtenus par polymérisation radicalaire des monomères de vinyl pyridine (substitués en position 2 ou 4 du noyau pyridine) de structure suivante :

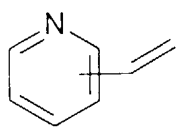

Les copolymères peuvent être par exemple des copolymères de vinylpyridine comportant au moins 5% de motifs vinylpyridine avec des monomères choisis parmi les motifs : vinylpyrrolidinone, acide acrylique et acrylamide.

(D) Les produits d'addition de monomères 1-vinylimidazole répondant à la formule (1):

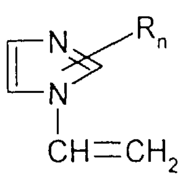

(1)

dans laquelle les radicaux R, identiques ou différents, représentent H, ou un radical alkyle en $C_1$-$C_6$, saturé ou insaturé, linéaire ou cyclique,
n est un entier allant de 1 à 3
avec les polyalkylènepolyamines et leurs dérivés (A)(i) à (A)(ix).

Parmi les dérivés de formule (1) utilisables on peut citer par exemple le 2-méthyl 1-vinyl imidazole, le 2-benzyl 1-vinyl imidazole.

Ces produits sont connus de l'homme du métier : Leur préparation est décrite par exemple dans la demande de brevet WO 94/29422.

(E) Les polymères à base d'acides aminés à chaîne latérale basique sont préférentiellement choisis parmi les protéines et les peptides comprenant au moins 5%, avantageusement au moins 10% d'acides aminés choisis parmi l'histidine, la lysine, l'arginine.
Parmi ces polymères on peut citer par exemple les polylysines, les polyhistidines.

(F) Les dérivés réticulés des polymères (A)(i) à (A)(ix), (B), (C) et (D). Parmi les réticulants utilisables, on peut citer les dérivés halo- hydrin-, glycidyl, aziridino-, isocyanate ; de tels réticulants ainsi que leurs méthodes d'utilisation sont bien connus de l'homme du métier. Parmi les plus connus on peut citer: l'épichlorhydrine, les $\alpha,\omega$-bis-(chlorhydrin) polyalkylène glycoléthers, les $\alpha,\omega$-dichloroalkane comme par exemple le 1,2-dichloroéthane, le 1,2-dichloropropane, le 1,3-dichloropropane, le 1,4-dichlorobutane, et le 1,6-dichlorohexane ; de tels réticulants

et leur utilisation pour réticuler des dérivés de polyéthylène imine sont décrits dans WO 94/12560.

De préférence, on choisit dans la mise en oeuvre de la présente invention des polymères polyaminés comprenant au moins 5% d'amines tertiaires, avantageusement au moins 10% de fonctions amines tertiaires, et encore plus préférentiellement au moins 20%.

Selon l'invention, le polymère polyaminé est choisi avantageusement parmi :

(A)

(i) les polyéthylèneimines hyperbranchées,
(ii) les dérivés alkylés de polyéthylèneimine;
(iii) les produits d'addition d'acides alkylcarboxyliques sur la polyéthylèneimine ;
(iv) les produits d'addition de cétones et d'aldéhydes sur la polyéthylèneimine;
(v) les produits d'addition d'isocyanates et d'isothiocyanates sur la polyéthylèneimine ;
(vi) les produits d'addition d'oxyde d'alkylène ou de polymères blocs polyoxyde d'alkylène sur la polyéthylèneimine;
(vii) les dérivés quaternisés de la polyéthylèneimine ;
(viii) les produits d'addition d'une silicone sur les polyéthylèneimine ;
(ix) les copolymères d'acide dicarboxylique et de polyéthylèneimine ;

(B) les polyvinylimidazoles .

Encore plus préférentiellement, le polymère polyaminé est choisi parmi :
(A) (i) les polyéthylèneimines hyperbranchées. De préférence, on choisit des polyéthylèneimines comprenant au moins 5% d'amines tertiaires, avantageusement au moins 10% de fonctions amines tertiaires, et encore plus préférentiellement au moins 20%.

De façon avantageuse, le polymère polyaminé est introduit dans les compositions selon l'invention sous une forme neutralisée.

D'une manière générale, le ou les polymères polyaminés peuvent être présents dans les compositions conformes à l'invention à des teneurs qui sont généralement comprises entre 0,01 % et 50 % en poids, et de préférence à des teneurs comprises entre 0,1 % et 30 % en poids, par rapport au poids total de la composition.

L'homme du métier saura ajuster par de simples essais les proportions de polymère polyaminé par rapport au dérivé du dibenzoylméthane, et éventuellement par rapport aux autres composants de la composition, en particulier la 1,3,5-triazine. En effet, les proportions optimales des différents constituants peuvent varier, par exemple en fonction du poids moléculaire du polymère, du taux d'amines et/ou du taux d'amines tertiaires dans ce polymère.

Comme indiqué précédemment, les dérivés du dibenzoylméthane utilisables selon la présente invention sont ceux répondant à la formule (V) suivante :

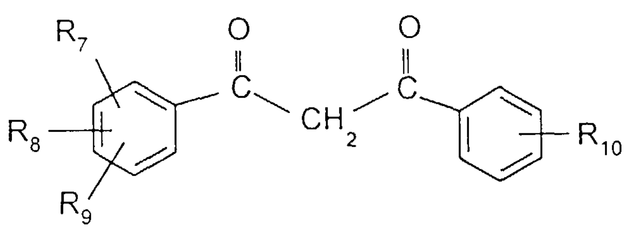
(V)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$.

Selon la présente invention, on peut bien entendu mettre en oeuvre un ou plusieurs dérivés du dibenzoylméthane.

Parmi les dérivés du dibenzoylméthane utilisables selon la présente invention, on peut notamment citer, de manière non limitative :

- le 2-méthyldibenzoylméthane,
- le 4-méthyldibenzoylméthane,
- le 4-isopropyldibenzoylméthane,
- le 4-tert.-butyldibenzoylméthane,

- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

Ces produits sont déjà bien connus et sont décrits notamment dans les documents FR-A-2 326 405, FR-A-2 440 933 et EP-A- 0 114 607 précités.

Parmi les dérivés du dibenzoylméthane mentionnés ci-dessus, on préfère tout particulièrement, selon la présente invention, mettre en oeuvre le 4-ter-butyl-4'-méthoxy dibenzoylméthane, notamment celui proposé à la vente sous la dénomination commerciale de "PARSOL 1789" par la Société GIVAUDAN, ce filtre répondant donc à la formule développée suivante :

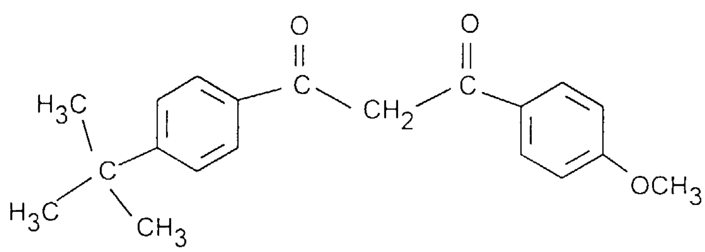

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

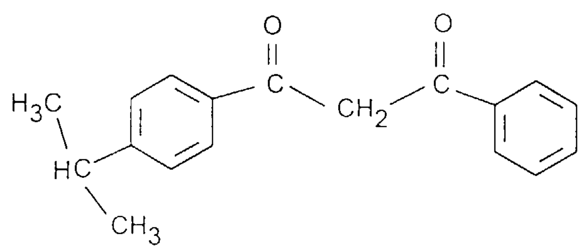

Les dérivés de dibenzoylméthane peuvent être présents dans les compositions de l'invention à une teneur allant de 0,2 % à 15 % en poids, par rapport au poids total de la composition. De préférence, cette teneur va de 0,2 % à 10 %.

Ainsi, lorsqu'on ajoute en quantité suffisante un polymère polyaminé à une composition antisolaire contenant un dérivé de dibenzoylméthane, en particulier du 4-tert-butyl-4'-méthoxydibenzoylméthane, on observe une augmentation de la stabilité dudit dérivé de dibenzoylméthane à la lumière, et donc une amélioration de l'efficacité de la composition antisolaire au cours du temps. De façon parallèle, on observe une diminution de l'apparition de dérivés peroxydés des dérivés dibenzoylméthane décrits ci-dessus. L'invention a donc également pour objet l'utilisation d'un polymère polyaminé tel que défini ci-dessus comme agent anti-oxydant vis-à-vis des dérivés du dibenzoylméthane.

Les dérivés de 1,3,5-triazine utilisables dans le cadre de la présente invention sont choisis parmi ceux répondant à la formule (I) suivante :

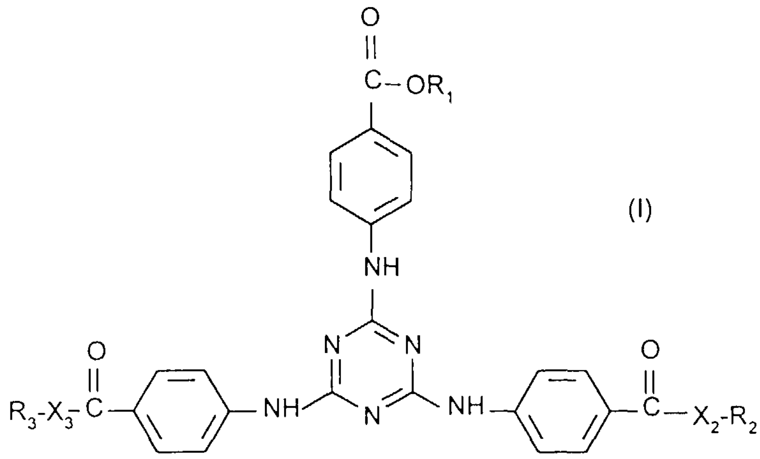

(I)

dans laquelle :

- $X_2$ et $X_3$, identiques ou différents, représentent l'oxygène ou le radical -NH-;
- $R_1$, $R_2$ et $R_3$, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en $C_1$-$C_{18}$; un radical cycloalkyle en $C_5$-$C_{12}$ éventuellement substitué par un ou plusieurs radicaux alkyles en $C_1$-$C_4$; un radical polyoxyéthyléné comprenant de 1 à 6 unités d'oxyde d'éthylène et dont le groupe OH terminal est méthylé; un radical de formule (II), (III) ou (IV) suivantes :

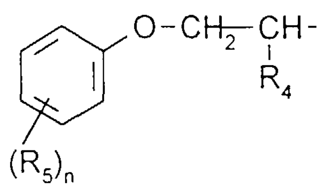

(II)

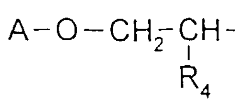

(III)

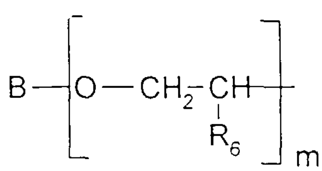

(IV)

dans lesquelles :

- $R_4$ est l'hydrogène ou un radical méthyle;
- $R_5$ est un radical alkyle en $C_1$-$C_9$;
- n est un nombre entier allant de 0 à 3;

- m est un nombre entier allant de 1 à 10;

- A est un radical alkyle en $C_4$-$C_8$ ou un radical cycloalkyle en $C_5$-$C_8$;

- B est choisi parmi : un radical alkyle linéaire ou ramifié en $C_1$-$C_8$; un radical cycloalkyle en $C_5$-$C_8$; un radical aryle éventuellement substitué par un ou plusieurs radicaux alkyles en $C_1$-$C_4$;

- $R_6$ est l'hydrogène ou un radical méthyle.

Bien entendu, dans la définition ci-dessus, lorsque $X_2$ et/ou $X_3$ représentent un radical - NH-, alors le ou les radicaux $R_2$ et/ou $R_3$ correspondants sont différents d'un métal alcalin ou d'un radical ammonium.

Une première famille plus particulièrement préférée de dérivés de 1,3,5-triazine est celle, notamment décrite dans le document EP-A-0 517 104, des 1,3,5-triazines répondant à la formule (I) ci-dessus et présentant l'ensemble des caractéristiques suivantes :

- $X_2$ et $X_3$ sont identiques et représentent l'oxygène;
- $R_1$ est choisi parmi : un radical cycloalkyle en $C_5$-$C_{12}$ éventuellement substitué par un ou plusieurs radicaux alkyles en $C_1$-$C_4$; un radical de formule (II), (III) ou (IV) ci-dessus dans lesquelles :

  - B est un radical alkyle en $C_1$-$C_4$ ;
  - $R_6$ est le radical méthyle ;

- $R_2$ et $R_3$, identiques ou différents, sont choisis parmi : l'hydrogène ; un métal alcalin ; un radical ammonium éventuellement substitué par un ou plusieurs radicaux alkyles ou hydroxyalkyles ; un radical alkyle linéaire ou ramifié en $C_1$-$C_{18}$ ; un radical cycloalkyle en $C_5$-$C_{12}$ éventuellement substitué par un ou plusieurs radicaux alkyles en $C_1$-$C_4$ ; un radical de formule (II), (III) ou (IV) ci-dessus dans lesquelles :

  - B est un radical alkyle en $C_1$-$C_4$;
  - $R_6$ est le radical méthyle.

Une deuxième famille préférée de dérivés de 1,3,5-triazine selon l'invention est celle, notamment décrite dans le document EP-A-0570838, des 1,3,5-triazines répondant à la formule (I) et présentant l'ensemble des caractéristiques suivantes :

- $X_3$ est le radical -NH-;
- $R_3$ est choisi parmi : un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- $R_1$ est choisi parmi : l'hydrogène ; un métal alcalin ; un radical ammonium ; un radical de formule (IV) ; un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- si $X_2$ est le radical -NH-, alors $R_2$ est choisi parmi : un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- si $X_2$ est l'oxygène, alors $R_2$ est choisi parmi l'hydrogène ; un métal alcalin ; un radical ammonium ; un radical de formule (IV) ; un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$.

Une 1,3,5-triazine particulièrement préférée de cette deuxième famille est celle répondant à la formule suivante :

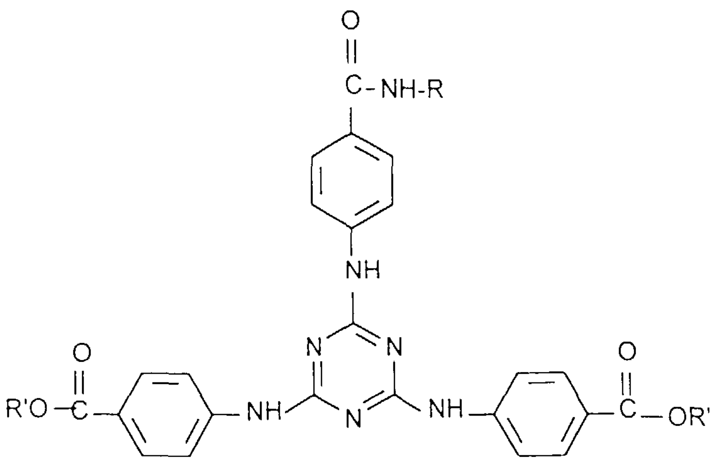

dans laquelle R' désigne un radical éthyl-2 hexyle et R désigne un radical tert. butyle.

Une troisième famille préférée de composés est celle, notamment décrite dans le document US 4,724,137, des 1,3,5-triazines répondant à la formule (I) et présentant l'ensemble des caractéristiques suivantes :

- $X_2$ et $X_3$ sont identiques et représentent l'oxygène;
- $R_1$, $R_2$ et $R_3$ sont identiques et représentent un radical alkyle en $C_6$-$C_{12}$ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine qui est un filtre connu en soi, actif dans l'UV-B, se présentant sous une forme solide, et qui est vendu notamment sous la dénomination commerciale de "UVINUL T 150" par la Société BASF. Ce produit répond à la formule suivante :

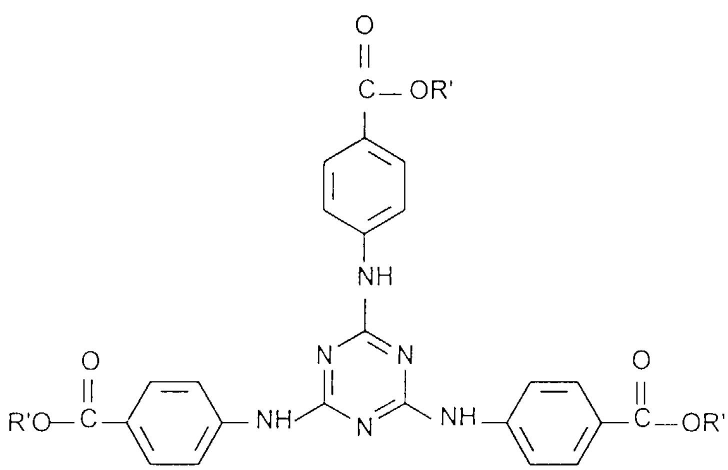

dans laquelle R' désigne un radical 2-éthyl hexyle.

Le ou les dérivés de 1,3,5-triazine sont généralement présents dans les compositions de l'invention à une teneur pouvant aller de 0,5 % à 20 %, de préférence de 1 % à 10 % en poids, par rapport au poids total de la composition.

Avantageusement, les compositions selon l'invention contiennent en outre au moins un nanopigment d'oxyde métallique.

Selon l'invention, on entend par "nanopigment" un pigment dont la taille des particules primaires a une dimension moyenne inférieure à 100 nm et de préférence comprise entre 5 et 50 nm.

Les oxydes métalliques sont choisis parmi les oxydes de titane, de zinc, de cérium, de zirconium ou leurs mélanges.

Les nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple, dans Cosmetics & Toiletries, Février 1990, vol.105, p.53-64, tels que des aminoacides, de la cire d'abeille, des acides gras, des alcools gras, des tensioactifs anioniques, des lécithines, des sels de sodium, potassium, zinc, fer ou aluminium d'acides gras, des silicones, des protéines (collagène, élastine), des alcanolamines, des oxydes de silicium, des oxydes métalliques ou de l'hexamétaphosphate de sodium, de polyols, d'huiles perfluorées.

Les pigments enrobés sont plus particulièrement des oxydes de titane enrobés :

- de silice tels que le produit "SUNVEIL" de la société IKEDA,
- de silice et d'oxyde de fer tel que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "UV TITAN M212" de la société KEMIRA,
- d'alumine et de stéarate d'aluminium tels que le produit "MICROTITANIUM DIOXIDE MT 100 T" de la société TAYCA,
- d'alumine et de laurate d'aluminium tels que le produit "MICROTITANIUM DIOXIDE MT 100 F" de la société TAYCA,
- d'oxyde de zinc et de stéarate de zinc tels que le produit "BR 351" de la société TAYCA,
- de silice, d'alumine et de silicone tels que les produits "MICROTITANIUM DIOXIDE MT SAS" de la société TAYCA,
- de silice, d'alumine et de perfluoropolyméthylisopropyléther tel que le produit "TiO2 VF-25-33" de la société TOSHIKI,
- de silice, d'alumine, de stéarate d'aluminium et de silicone tels que le produit "STT-30-DS" de la société TITAN KOGYO,
- d'alumine et de silicone tels que les produits "TIPAQUE TTO-55 (S)" de la société ISHIHARA, et "UV TITAN M262" de la société KEMIRA,
- de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

On peut utiliser également les associations de nanopigments d'oxyde de titane enrobés ou non enrobés rendus dispersibles dans l'eau par un traitement hydrophile ou dispersibles dans les huiles par un traitement hydrophobe telles que celles décrites dans la demande de brevet européen 456 460.

On peut également citer les mélanges d'oxydes métallique, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobés d'alumine, de silice et de silicone tel que le produit "M 261" vendu par la société KEMIRA ou enrobés d'alumine, de silice et de glycérine tel que le produit "M 211" vendu par la société KEMIRA.

Les oxydes de titane non enrobés sont par exemple vendus par la société TAYCA sous les dénominations commerciales "MICROTITANIUM DIOXIDE MT 500 B" ou "MICROTITANIUM DIOXIDE MT 600 B", par la société DEGUSSA sous la dénomination "P 25", par la société WACKHERR sous la dénomination "Oxyde de titane transparent PW", par la société MIYOSHI KASEI sous la dénomination "UFTR", par la société TOMEN sous la dénomination "ITS".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25", par la société IKEDA sous la dénomination "MZO-25" ou par la société SUNSMART sous la dénomination "Z-COTE".

L'oxyde de cérium non enrobé est vendu sous la dénomination "COLLOIDAL CERIUM OXIDE" par la société RHONE POULENC.

Selon l'invention, les nanopigments d'oxyde de titane, enrobés ou non enrobés, sont particulièrement préférés.

La concentration en nanopigments d'oxydes métalliques dans les compositions cosmétiques selon l'invention est comprise entre 0,1 et 20 % en poids par rapport au poids total de la composition, et de préférence entre 0,25 et 15 %.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles, autres bien sûr que les filtres mentionnés ci-avant. Ces filtres complémentaires peuvent être notamment choisis parmi les dérivés cinnamiques, les dérivés salicyliques, les dérivés du benzylidène camphre, les dérivés de benzimidazole, les dérivés de triazine autres que ceux ci-avant mentionnés, les dérivés de la benzophénone, les dérivés de $\beta,\beta'$-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments autres que les nanopigments décrits ci-dessus.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (en particulier les filtres complémentaires) et/ou leurs quantités de manière telle que les propriétés avantageuses attachées intrinsèquement à l'association ternaire conforme à l'invention ne soient pas, ou substantiellement pas, altérées par la ou les adjonctions envisagées et de manière telle que les compositions de l'invention présentent de bonnes propriétés cosmétiques.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Un exemple concret, mais nullement limitatif, illustrant l'invention, va maintenant être donné.

**ESSAIS** :

Dans les essais décrits ci-dessous, nous avons utilisé la polyéthylèneimine (PEI) de poids moléculaire 700 commercialisée par la société Aldrich.

Les pourcentages de composants dans les formules sont donnés en poids par rapport au poids total de la formule.

**ESSAI 1 : Test EX-VIVO d'inhibition de la photo-peroxydation du 4-tert-butyl-4'-méthoxydibenzoylméthane par la polyéthylèneimine sous UVA**

On applique sur un premier filtre circulaire de $17cm^2$ un film mince de produit témoin (formule A : 4-tert-butyl-4'-méthoxydibenzoylméthane) à raison d'environ 3 $mg/cm^2$. On applique sur un second filtre le produit contenant la polyéthylènimine (produit B : 4-tert-butyl-4'-méthoxydibenzoylméthane + PEI). Les filtres sont ensuite irradiés sous 20 joules UVA/cm2 à l'aide d'un appareil Biotronic 360.

Les peroxydes de 4-tert-butyl-4'-méthoxydibenzoylméthane sont extraits des filtres, à l'aide de 5 ml d'éthanol. Les peroxydes sont dosés dans ces extraits par HPLC.

Les résultats sont exprimés en inhibition de la péroxydation du 4-tert-butyl-4'-méthoxydibenzoylméthane :

$$\% \text{ inhibition} = \frac{\text{ROOH(formuleA) - ROOH(formuleB)}}{\text{ROOH(formuleA)}} \text{ X } 100$$

ROOH représente la quantité de peroxydes de 4-tert-butyl-4'-méthoxydibenzoylméthane dans la formule (picomoles de péroxydes en équivalent $H_2O_2$ par mg de produit extrait).

La composition des formules A et B est la suivante :

| Nom CTFA | Formule A | Formule B |
|---|---|---|
| Cetyl alcohol | 5% | 5% |
| Gyceryl stearate | 3% | 3% |
| P.E.G. 50 stearate | 3% | 3% |
| Mineral oil | 20% | 20% |
| Caprylic/Capric triglycerides | 3% | 3% |
| 4-tert-butyl-4'-méthoxydibenzoylméthane | 1% | 1% |
| Water | QSP 100% | QSP 100% |
| Polyéthylènimine | 0% | 2% |

On obtient les résultats suivants :

| Formule | Péroxydes en pmoles/mg |
|---|---|
| Formule A | 2600 |
| Formule B | 1010 |

Soit une inhibition de 60% de la photoperoxydation du 4-tert-butyl-4'-méthoxydibenzoylméthane par rapport à la formule A.

**ESSAI 2 : Test de photostabilisation du 4-tert-butyl-4'-méthoxy-dibenzoylméthane par la polyéthylèneimine sous UVA**

Des films de formule anti-solaire sont préparés par étalement manuel de la formule à raison de 2mg/cm$^2$ sur un support en polyméthacrylate de méthyle dépoli (PMMA) ou de verre dépoli.

Les échantillons ainsi préparés sont ensuite exposés pendant 2H30 ou 4H30, au rayonnement d'un Sun-test HERAEUS (source: Arc long Xénon 1,8 kW), dans une enceinte dont la température est régulée aux environs de 35 à 40°C, afin de simuler une irradiation UV naturelle.
Cette exposition correspond à environ 30 J/cm$^2$ UVA ( 2H30) ou 54 J/cm$^2$ UVA (4H30).

Après exposition, les filtres UV sont extraits par 63 ml d'éthanol par échantillon.
Les solutions obtenues sont analysées par Spectrophotométrie. On mesure la densité optique de cette solution au λmax du filtre.
Parallèlement, on extrait et on analyse selon le même protocole les filtres d'un échantillon de formule témoin (même composition) appliqué sur le support de PMMA dépoli ou de verre dépoli, mais n'ayant pas subi d'irradiation UV .

Le taux de filtres résiduels après irradiation est donné, pour chacun des filtres de la formule, par le rapport de la densité optique dans l'échantillon irradié à sa densité optique dans l'échantillon non irradié (échantillon témoin).

La composition des formules testées est la suivante :

| | A' | B' |
|---|---|---|
| -Stéarate de polyéthylène glycol (40 OE) (commercialisé par ICI sous la marque Myrj 52) | 4 | 4 |
| -Tristéarate de sorbitane (commercialisé par ICI sous la marque Span 65) | 0.9 | 0.9 |
| -Alcool cétylique | 4 | 4 |
| -Stéarate de glycerol codex (STEARINERIE DUBOIS) | 3.3 | 3.3 |
| -Benzoate d'alcools $C_{12}/C_{15}$ (commercialisé par WITCO sous la marque Witconol) | 10 | 10 |
| -Huile de vaseline | 5 | 5 |
| - 4-tert-butyl-4'-méthoxy-dibenzoylméthane | 2 | 2 |
| -Polyéthylènimine | / | 8 |
| -Acide chlorhydrique | / | qs neutralisation |
| -Glycérine | 6 | 6 |
| -EDTA | 0.1 | 0.1 |
| -Conservateurs | qs | qs |
| -Eau déminéralisée qsp | 100 g | 100 g |

On irradie par 30 J/cm$^2$, on obtient les résultats suivants

| | formule A' | formule B' |
|---|---|---|
| % filtre résiduel mesuré à 358 nm | 27 ± 2 | 40 ± 3 |

**ESSAI 3 : Test de photostabilisation du 4-tert-butyl-4'-méthoxy-dibenzoylméthane en présence de 2,4,6-tris[p-(2'-éthylhexyl-1'-oxycarbonyl)anilino]-1,3,5-triazine par la polyéthylèneimine sous UVA**

On applique le même protocole qu'à l'essai 2 aux formules suivantes :

| | A" | B" | C" | D" |
|---|---|---|---|---|
| -Mélange d'alcool cétylstéarylique et d'alcool cétylstéarylique oxyéthyléné(33 OE)80/20 (commercialisé par la société TENSIA sous le nom de marque Dehsconet 390) | 7 | 7 | 7 | 7 |
| -Mélange de mono et distéarate de glycérol (commercialisé par la société ISP sous le nom de marque Cerasynth SD) | 2 | 2 | 2 | 2 |
| -Alcool cétylique | 1.5 | 1.5 | 1.5 | 1.5 |
| -Polydiméthylsiloxane (commercialisé par la société DOW CORNING sous le nom de marque DC200 Fluid) | 1.5 | 1.5 | 1.5 | 1.5 |
| -Benzoate d'alcools $C_{12}/C_{15}$ (commercialisé par la société WITCO sous le nom de marque Witcconol TN) | 15 | 15 | 15 | 15 |
| - 2,4,6-tris[p-(2'-éthylhexyl-1'-oxycarbonyl)anilino]-1,3,5-triazine | 1.5 | | 1.5 | 1.5 |
| - 4-tert-butyl-4'-méthoxy-dibenzoylméthane | / | 0.5 | 0.5 | 0.5 |
| -EDTA | / | 0.1 | 0.1 | 0.1 |
| -Polyéthylènimine | / | / | / | 4 |
| -Acide chlorhydrique | / | / | / | qs pH:7 |
| -Conservateurs | qs | qs | qs | qs |
| -Eau déminéralisée qsp | 100 g | 100 g | 100 g | 100 g |

On irradie par 54 $J/cm^2$. On obtient les résultats suivants :

| | % filtre [2,4,6-tris[p-(2'-éthylhexyl-1'-oxycarbonyl)anilino]-1,3,5-triazine] résiduel mesuré à 312 nm | % filtre [4-tert-butyl-4'-méthoxy-dibenzoylméthane] résiduel mesuré à 358 nm |
|---|---|---|
| formule A" | 102 ± 9 | - |
| formule B" | - | 40 ± 4 |
| formule C" | 89 ± 3 | 39 ± 5 |
| formule D" | 97 ± 4 | 60 ± 12 |

**Revendications**

**1.** Composition cosmétique et/ou dermatologique comprenant, dans un support cosmétiquement et/ou dermatologiquement acceptable :

a) au moins un polymère polyaminé choisi parmi :

(A) une polyalkylène polyamine ou un dérivé de polyalkylène polyamine choisi parmi :

(i) les polyalkylène polyamines comprenant de 7 à 20000 motifs de répétition ;
(ii) les dérivés alkylés de polyalkylène polyamines ;
(iii) les produits d'addition d'acides alkylcarboxyliques sur les polyalkylènepolyamines ;
(iv) les produits d'addition de cétones et d'aldéhydes sur les polyalkylènepolyamines ;
(v) les produits d'addition d'isocyanates et d'isothiocyanates sur les polyalkylènepolyamines ;
(vi) les produits d'addition d'oxyde d'alkylène ou de polymères blocs polyoxyde d'alkylène sur les polyalkylènepolyamines ;
(vii) les dérivés quaternisés de polyalkylène polyamines ;
(viii) les produits d'addition d'une silicone sur les polyalkylènepolyamines ;
(ix) un copolymère d'acide dicarboxylique et de polyalkylène polyamines ;

(B) les polyvinylimidazoles ;

(C) les polyvinylpyridines ;

(D) les produits d'addition de monomères 1-vinylimidazole de formule (I) :

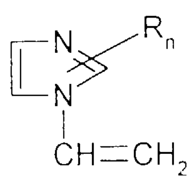

(I)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(ix) ;

(E) les polymères à base d'acides aminés à chaîne latérale basique ;

(F) les dérivés réticulés des polymères (A)(i) à (A)(ix), (B) (C) (D) et (E) ;

b) au moins un dérivé de dibenzoylméthane répondant à la formule (V) suivante :

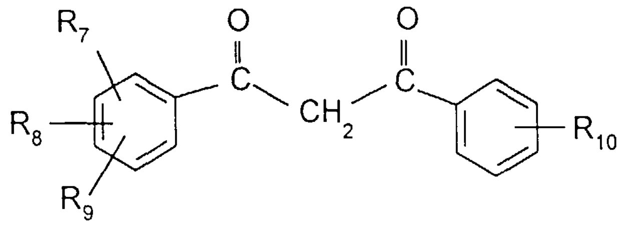

(V)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$.

**2.** Composition selon la revendication précédente, caractérisée par le fait que le polymère polyaminé est choisi parmi ceux comprenant au moins 5% de fonctions amines tertiaires, avantageusement au moins 10% de fonctions amines tertiaires, et encore plus préférentiellement au moins 20%.

**3.** Composition selon l'une quelconque des revendications précédentes, caractérisée par le fait que le polymère polyaminé est choisi parmi :

(A)

(i) les polyéthylèneimines hyperbranchées,
(ii) les dérivés alkylés de polyéthylèneimine ;
(iii) les produits d'addition d'acides alkylcarboxyliques sur la polyéthylèneimine ;
(iv) les produits d'addition de cétones et d'aldéhydes sur la polyéthylèneimine ;
(v) les produits d'addition d'isocyanates et de thioisocyanates sur la polyéthylèneimine ;
(vi) les produits d'addition d'oxyde d'alkylène ou de polymères blocs polyoxyde d'alkylène sur la polyéthylèneimine ;
(vii) les dérivés quaternisés de polyéthylèneimine ;
(viii) les produits d'addition d'une silicone sur la polyéthylèneimine ;
(ix) un copolymère d'acide dicarboxylique et de polyéthylèneimine ;

(B) les polyvinylimidazoles .

**4.** Composition selon l'une quelconque des revendications précédentes, caractérisée par le fait que le polymère polyaminé est choisi parmi les polyéthylèneimines hyperbranchées.

**5.** Composition selon l'une quelconque des revendications 1 à 4, caractérisée par le fait que le polymère polyaminé est présent dans la composition à une teneur allant de 0,5 % à 20 % en poids, par rapport au poids total de la composition, de préférence de 1 % à 10 % en poids, par rapport au poids total de la composition.

**6.** Composition selon l'une quelconque des revendications précédentes, caractérisée par le fait que dérivé de dibenzoylméthane est choisi parmi :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,

- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

**7.** Composition selon l'une quelconque des revendications précédentes, caractérisée par le fait que le dérivé de dibenzoylméthane est le 4-tert-butyl-4'-méthoxydibenzoylméthane.

**8.** Composition selon l'une quelconque des revendications précédentes, caractérisée par le fait que le dérivé de dibenzoylméthane est présent dans la composition à une teneur allant de 0,2 % à 15 % en poids, par rapport au poids total de la composition, de préférence, de 0,2 % à 10 % en poids, par rapport au poids total de la composition.

**9.** Composition cosmétique selon l'une quelconque des revendications précédentes, caractérisée en ce qu'elle comprend en outre : au moins un dérivé de 1,3,5-triazine répondant à la formule (I) suivante :

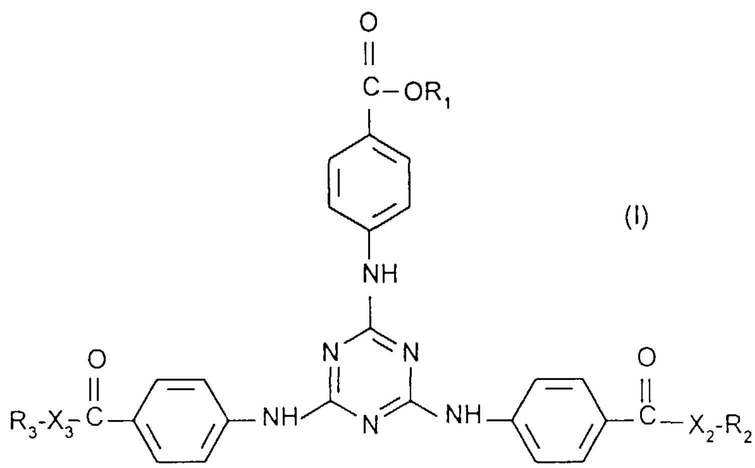

(I)

dans laquelle :

- $X_2$ et $X_3$, identiques ou différents, représentent l'oxygène ou le radical -NH-;
- $R_1$, $R_2$ et $R_3$, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en $C_1$-$C_{18}$; un radical cycloalkyle en $C_5$-$C_{12}$ éventuellement substitué par un ou plusieurs radicaux alkyles en $C_1$-$C_4$; un radical polyoxyéthyléné comprenant de 1 à 6 unités d'oxyde d'éthylène et dont le groupe OH terminal est méthylé; un radical de formule (II), (III) ou (IV) suivantes :

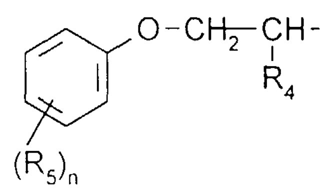

(II)

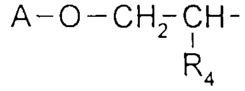

(III)

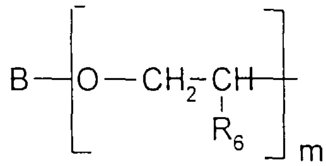

(IV)

dans lesquelles :

- $R_4$ est l'hydrogène ou un radical méthyle;
- $R_5$ est un radical alkyle en $C_1$-$C_9$;
- n est un nombre entier allant de 0 à 3;
- m est un nombre entier allant de 1 à 10;
- A est un radical alkyle en $C_4$-$C_8$ ou un radical cycloalkyle en $C_5$-$C_8$;
- B est choisi parmi : un radical alkyle linéaire ou ramifié en $C_1$-$C_8$; un radical cycloalkyle en $C_5$-$C_8$; un radical aryle éventuellement substitué par un ou plusieurs radicaux alkyles en $C_1$-$C_4$;
- $R_6$ est l'hydrogène ou un radical méthyle.

**10.** Composition selon la revendication précédente, caractérisée par le fait que le dérivé de 1,3,5-triazine est choisi parmi ceux de formule (I) présentant l'ensemble des caractéristiques suivantes :

- $X_2$ et $X_3$ sont identiques et représentent l'oxygène ;
- $R_1$ est choisi parmi : un radical cycloalkyle en $C_5$-$C_{12}$ éventuellement substitué par un ou plusieurs radicaux alkyles en $C_1$-$C_4$; un radical de formule (II), (III) ou (IV) dans lesquelles :
  - B est un radical alkyle en $C_1$-$C_4$ ;
  - $R_6$ est le radical méthyle ;
- $R_2$ et $R_3$, identiques ou différents, sont choisis parmi : l'hydrogène ; un métal alcalin ; un radical ammonium éventuellement substitué par un ou plusieurs radicaux alkyles ou hydroxyalkyles ; un radical alkyle linéaire ou ramifié en $C_1$-$C_{18}$ ; un radical cycloalkyle en $C_5$-$C_{12}$ éventuellement substitué par un ou plusieurs radicaux alkyles en $C_1$-$C_4$ ; un radical de formule (II), (III) ou (IV) dans lesquelles :
  - B est un radical alkyle en $C_1$-$C_4$ ;
  - $R_6$ est le radical méthyle.

**11.** Composition selon la revendication 9, caractérisée par le fait que le dérivé de 1,3,5-triazine est choisi parmi ceux de formule (I) présentant l'ensemble des caractéristiques suivantes :

- $X_2$ et $X_3$ sont identiques et représentent le radical -NH-;
- $R_3$ est choisi parmi : un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- $R_1$ est choisi parmi : l'hydrogène ; un métal alcalin ; un radical ammonium ; un radical de formule (IV) ; un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- $R_2$ est choisi parmi : un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$.

**12.** Composition selon la revendication 9, caractérisée par le fait que le dérivé de 1,3,5-triazine est choisi parmi ceux présentant l'ensemble des caractéristiques suivantes :

- $X_2$ est l'oxygène;
- $X_3$ est le radical -NH-;
- $R_3$ est choisi parmi : un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- $R_1$ est choisi parmi : l'hydrogène ; un métal alcalin ; un radical ammonium ; un radical de formule (IV); un radical alkyle linéaire ou ramifié en $C_1$-$C_{18}$ ; un radical cycloalkyle en $C_5$-$C_{12}$ éventuellement substitué avec

un ou plusieurs radicaux alkyles en $C_1$-$C_4$ ;

- $R_2$ est choisi parmi l'hydrogène ; un métal alcalin ; un radical ammonium ; un radical de formule (IV); un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$.

**13.** Composition selon la revendication 9, caractérisée par le fait que le dérivé de 1,3,5-triazine est celui répondant à la formule suivante :

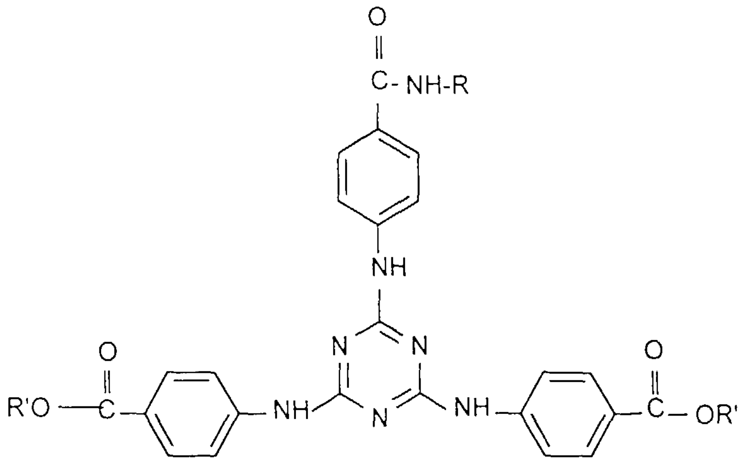

dans laquelle R' désigne un radical éthyl-2 hexyle et R désigne un radical ter. butyle.

**14.** Composition selon la revendication 9, caractérisée par le fait que le dérivé de 1,3,5-triazine est choisi parmi ceux présentant l'ensemble des caractéristiques suivantes :

- $X_2$ et $X_3$ sont identiques et représentent l'oxygène;
- $R_1$, $R_2$ et $R_3$ sont identiques et représentent un radical alkyle en $C_6$-$C_{12}$ ou un radical polyoxyéthyléné comprenant de 1 à 6 unités d'oxyde d'éthylène et dont le groupe OH terminal est méthylé.

**15.** Composition selon la revendication 9, caractérisée par le fait que le dérivé de 1,3,5-triazine est celui répondant à la formule suivante :

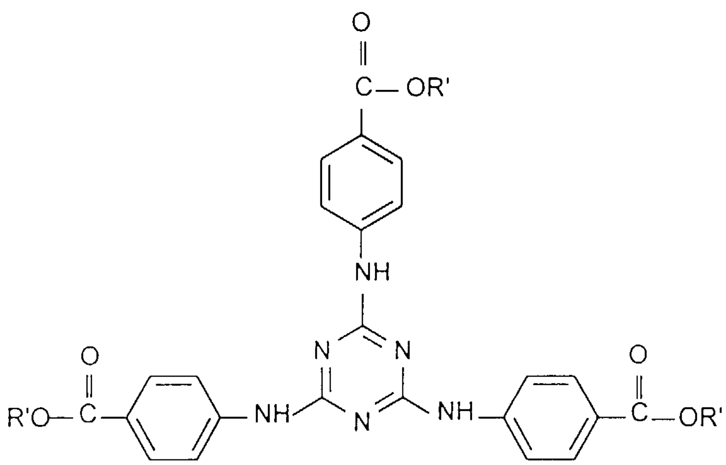

dans laquelle R' désigne un radical 2-éthyl hexyle.

**16.** Composition selon l'une quelconque des revendications 9 à 15, caractérisée par le fait que le dérivé de 1,3,5-triazine est présent dans la composition à une teneur allant de 0,5 % à 20 % en poids, par rapport au poids total de

la composition, de préférence de 1 % à 10 % en poids, par rapport au poids total de la composition.

**17.** Composition selon l'une quelconque des revendications précédentes, caractérisée par le fait que le polymère polyaminé est introduit sous une forme neutralisée.

**18.** Composition cosmétique selon l'une quelconque des revendications précédentes, caractérisée en ce qu'elle comprend en outre au moins un nanopigment.

**19.** Composition cosmétique selon la revendication précédente, caractérisée en ce que le nanopigment est un nanopigment d'oxyde de titane.

**20.** Composition cosmétique selon l'une quelconque des revendications 18 et 19 précédentes, caractérisée en ce que la concentration en nanopigments d'oxydes métalliques dans la composition est comprise entre 0,1 et 20 % en poids par rapport au poids total de la composition, et de préférence entre 0,25 et 15 %.

**21.** Composition selon l'une quelconque des revendications précédentes, caractérisée par le fait qu'elle est sous la forme d'une émulsion huile-dans-eau.

**22.** Utilisation d'un polymère polyaminé tel que défini à l'une quelconque des revendications 1 à 4 comme agent antioxydant vis-à-vis des dérivés du dibenzoylméthane.

**23.** Utilisation d'un polymère polyaminé tel que défini à l'une quelconque des revendications 1 à 4 dans, ou pour la fabrication de, compositions cosmétiques et/ou dermatologiques contenant un dérivé de dibenzoylméthane tel que défini à l'une quelconque des revendications 1, 6 et 7 en vue d'améliorer dans lesdites compositions la stabilité au rayonnement UV dudit dérivé de dibenzoylméthane.

**24.** Procédé pour améliorer la stabilité au rayonnement UV des compositions cosmétiques et/ou dermatologiques comprenant un dérivé de dibenzoylméthane tel que défini à l'une quelconque des revendications 1, 6 et 7, caractérisé par le fait qu'il consiste à introduire dans lesdites compositions une quantité efficace d'un polymère polyaminé tel que défini à l'une quelconque des revendications 1 à 4.

**25.** Utilisation d'un polymère polyaminé tel que défini à l'une quelconque des revendications 1 à 4 dans, ou pour la fabrication de, compositions cosmétiques et/ou dermatologiques contenant un dérivé de dibenzoylméthane, en association avec au moins un dérivé de 1,3,5-triazine tel que défini à l'une quelconque des revendications 9 à 15 en vue d'améliorer dans lesdites compositions la stabilité au rayonnement UV dudit dérivé de 1,3,5-triazine.

**26.** Procédé pour améliorer la stabilité au rayonnement UV d'une composition cosmétique et/ou dermatologique comprenant un dérivé de dibenzoylméthane et un dérivé de 1,3,5-triazine tels que définis à l'une quelconque des revendications 9 à 15, ledit procédé consistant à introduire dans ladite composition une quantité efficace d'un polymère polyaminé tel que défini à l'une quelconque des revendications 1 à 4.

**Patentansprüche**

**1.** Kosmetische und/oder dermatologische Zusammensetzung, die in einem kosmetisch und/oder dermatologisch akzeptablen Träger enthält:

a) mindestens ein Polyaminpolymer, das ausgewählt ist unter:

(A) den Polyalkylenpolyaminen oder Polyalkylenpolyaminderivaten, die ausgewählt sind unter:

(i) den Polyalkylenpolyaminen mit 7 bis 20 000 wiederkehrenden Einheiten;
(ii) den alkylierten Derivaten von Polyalkylenpolyaminen (A)(i);
(iii) den Additionsprodukten von Alkylcarbonsäuren und Polyalkylenpolyaminen (A)(i);
(iv) den Additionsprodukten von Ketonen und Aldehyden und Polyalkylenpolyaminen (A)(i);
(v) den Additionsprodukten von Isocyanaten und Isothiocyanaten und Polyalkylenpolyaminen (A)(i);
(vi) den Additionsprodukten von Alkylenoxid oder Polyalkylenoxid-Blockpolymeren und Polyalkylenpolyaminen (A)(i);

(vii) den quaternisierten Derivaten von Polyalkylenpolyaminen (A)(i);
(viii) den Additionsprodukten von Siliconen und Polyalkylenpolyaminen (A)(i);
(ix) den Copolymeren von Dicarbonsäuren und Polyalkylenpolyaminen (A)(i);

(B) den Polyvinylimidazolen;
(C) den Polyvinylpyridinen;
(D) den Additionsprodukten von 1-Vinylimidazolmonomeren der Formel (1):

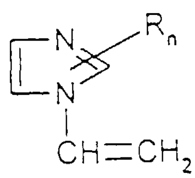

(1),

worin die Gruppen R, die identisch oder voneinander verschieden sind, H oder eine geradkettige oder cyclische, gesättigte oder ungesättigte $C_{1-6}$-Alkylgruppe und n eine ganze Zahl von 1 bis 3 bedeuten, mit den Polyalkylenpolyaminen (A)(i) bis (A)(ix);
(E) den Polymeren auf der Basis von Aminosäuren mit basischer Seitenkette;
(F) den vernetzten Derivaten von Polymeren (A)(i) bis (A)(ix), (B), (C), (D) und (E);

b) mindestens ein Dibenzoylmethanderivat der folgenden Formel (V),

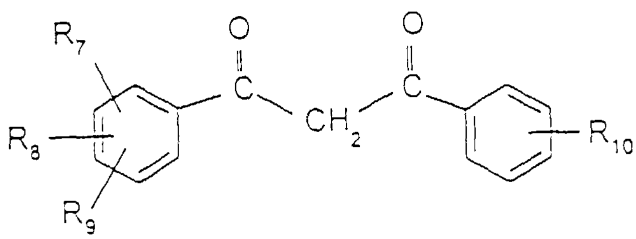

(V),

worin die Gruppen $R_7$, $R_8$, $R_9$ und $R_{10}$, die identisch oder voneinander verschieden sind, unabhängig voneinander Wasserstoff, Hydroxy, eine geradkettige oder verzweigte $C_{1-8}$-Alkylgruppe oder eine geradkettige oder verzweigte $C_{1-8}$-Alkoxygruppe bedeuten.

2. Zusammensetzung nach dem vorherigen Anspruch, dadurch gekennzeichnet, daß das Polyaminpolymer unter den Polymeren ausgewählt ist, die mindestens 5 % tertiäre Aminogruppe enthalten, vorteilhaft mindestens 10 % tertiäre Aminogruppen und noch bevorzugter mindestens 20 %.

3. Zusammensetzung nach einem der vorhergehenden Ansprüche, dadurch gekennzeichnet, daß das Polyaminpolymer ausgewählt ist unter;

(A)

(i) den hochverzweigten Polyethyleniminen;
(ii) den alkylierten Polyethyleniminderivaten;
(iii) den Additionsprodukten von Alkylcarbonsäuren und Polyethylenimin;
(iv) den Additionsprodukten von Ketonen und Aldehyden und Polyethylenimin;
(v) den Additionsprodukten von Isocyanaten und Isothiocyanaten und Polyethylenimin;
(vi) den Additionsprodukten von Alkylenoxid oder Polyalkylenoxid-Blockpolymeren und Polyethylenimin;
(vii) den quaternisierten Derivaten von Polyethylenimin;
(viii) den Additionsprodukten von Siliconen und Polyethylenimin;
(ix) den Copolymeren von Dicarbonsäuren und Polyethylenimin;

(B) den Polyvinylimidazolen.

**4.** Zusammensetzung nach einem der vorhergehenden Ansprüche, dadurch gekennzeichnet, daß das Polyaminpolymer unter den hochverzweigten Polyethyleniminen ausgewählt ist.

**5.** Zusammensetzung nach einem der Ansprüche 1 bis 4, dadurch gekennzeichnet, daß das Polyaminpolymer in der Zusammensetzung in Mengenanteilen im Bereich von 0,5 bis 20 Gew.-% und vorzugsweise 1 bis 10 Gew.-%, bezogen auf das Gesamtgewicht der Zusammensetzung, vorliegt.

**6.** Zusammensetzung nach einem der vorhergehenden Ansprüche, dadurch gekennzeichnet, daß das Dibenzoylmethanderivat ausgewählt ist unter:

- 2-Methyl-dibenzoylmethan,
- 4-Methyl-dibenzoylmethen,
- 4-Isoproyl-dibenzoylmethan,
- 4-*tert*.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4-*tert*.-Butyl-4'-methoxy-dibenzoylmethan,
- 2-Methyl-5-isopropyl-4'-methoxy-dibenzoylmethan,
- 2-Methyl-5-*tert*.-butyl-4'-methoxy-dibenzoylmethan,
- 2,4-Dimethyl-4'-methoxy-dibenzoylmethan,
- 2,6-Dimethyl-4-*tert*.-butyl-4'-methoxy-dibenzoylmethan, und
- 4,4'-Dimethoxy-dibenzoylmethan.

**7.** Zusammensetzung nach einem der vorhergehenden Ansprüche, dadurch gekennzeichnet, daß das Dibenzoylmethanderivat das 4-*tert*.-Butyl-4'-methoxydibenzoylmethan ist.

**8.** Zusammensetzung nach einem der vorhergehenden Ansprüche, dadurch gekennzeichnet, daß das Dibenzoylmethanderivat in der Zusammensetzung in einem Mengenanteil von 0,2 bis 15 Gew.-%, bezogen auf das Gesamtgewicht der Zusammensetzung, und vorzugsweise 0,2 bis 10 Gew.-%, bezogen auf das Gesamtgewicht der Zusammensetzung, vorliegt.

**9.** Kosmetische Zusammensetzung nach einem der vorhergehenden Ansprüche, dadurch gekennzeichnet, daß sie ferner enthält:
mindestens ein 1,3,5-Triazinderivat der folgenden Formel (I):

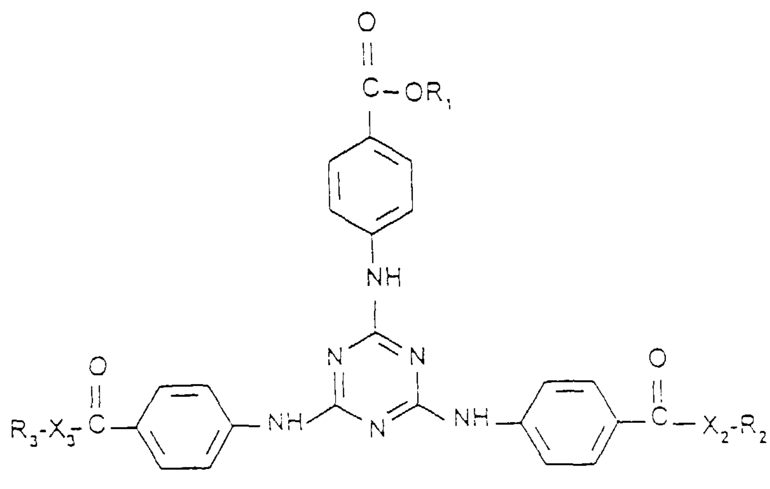

(I),

worin:

- die Gruppen $X_2$ und $X_3$, die identisch oder voneinander verschieden sind, Sauerstoff oder die Gruppe -NH- bedeuten; und

- die Gruppen $R_1$, $R_2$ und $R_3$, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten $C_{1-18}$-Alkylgruppe; einer $C_5$-$_{12}$-Cycloalkylgruppe, die gegebenenfalls mit einer oder mehreren $C_{14}$-Alkylgruppen substituiert ist; einer polyethoxylierten Gruppe, die 1 bis 6 Ethylenoxideinheiten aufweist und deren OH-Endgruppe methyliert ist; einer Gruppe der folgenden Formeln (II), (III) oder (IV):

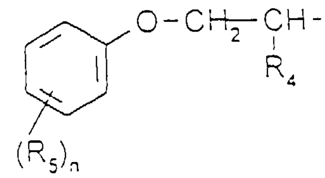

(II)

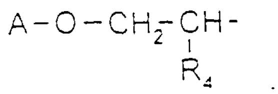

(III)

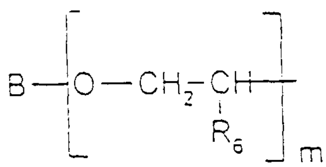

(IV),

worin bedeuten:

- $R_4$ Wasserstoff oder Methyl;
- $R_5$ eine $C_{1-9}$-Alkylgruppe;
- n Null oder eine ganze Zahl im Bereich von 1 bis 3;
- m eine ganze Zahl im Bereich von 1 bis 10;
- A eine $C_{4-8}$-Alkylgruppe oder eine $C_{5-8}$-Cycloalkylgruppe;
- B eine geradkettige oder verzweigte $C_{1-8}$-Alkylgrappe; eine $C_{5-8}$-Cycloalkylgruppe; eine Arylgruppe, die gegebenenfalls mit einer oder mehreren $C_{1-4}$-Alkylgruppen substituiert ist;
- $R_6$ Wasserstoff oder Methyl.

**10.** Zusammensetzung nach dem vorhergehenden Anspruch, dadurch gekennzeichnet, daß das 1,3,5-Triazinderivat unter den Verbindungen der Formel (I) ausgewählt ist, die alle folgenden Eigenschaften aufweisen:

- $X_2$ und $X_3$ sind identisch und bedeuten Sauerstoff;
- $R_1$ ist ausgewählt unter: einer $C_{5-12}$-Cycloalkylgruppe, die gegebenenfalls mit einer oder mehreren $C_{1-4}$-Alkylgruppen substituiert ist; einer Gruppe der Formel (II), (III) oder (IV), worin bedeuten:

  - B eine $C_{1-4}$-Alkylgruppe; und
  - $R_6$ die Methylgruppe;

- die Gruppen $R_2$ und $R_3$, die identisch oder verschieden sind, sind ausgewählt unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten $C_{1-18}$-Alkylgruppe; einer $C_{5-12}$-Cycloalkylgruppe, die gegebenenfalls mit einer oder mehreren $C_{1-4}$-Alkylgruppen substituiert ist; einer Gruppe der Formel (II), (III) oder (IV), worin bedeuten:

- B eine $C_{1-4}$-Alkylgruppe; und
- $R_6$ die Methylgruppe.

**11.** Zusammensetzung nach Anspruch 9, dadurch gekennzeichnet, daß das 1,3,5-Triazinderivat unter den Verbindungen der Formel (I) ausgewählt ist, die alle folgenden Eigenschaften aufweisen:

- $X_2$ und $X_3$ sind identisch und bedeuten die Gruppe -NH-;
- $R_3$ ist ausgewählt unter: einer geradkettigen oder verzweigten $C_{1-18}$-Alkylgruppe oder einer $C_{5-12}$-Cycloalkylgruppe die gegebenenfalls mit einer oder mehreren $C_{1-4}$-Alkylgruppen substituiert ist;
- $R_1$ ist ausgewählt unter: Wasserstoff; einem Alkalimetall; einer Ammoniumgruppe; einer Gruppe der Formel (IV); einer geradkettigen oder verzweigten $C_{1-18}$-Alkylgruppe; einer $C_{5-12}$-Cycloalkylgruppe, die gegebenenfalls mit einer oder mehreren $C_{1-4}$-Alkylgruppen substituiert ist;
- $R_2$ ist ausgewählt unter: einer geradkettigen oder verzweigten $C_{1-18}$-Alkylgruppe oder einer $C_{5-12}$-Cycloalkylgruppe die gegebenenfalls mit einer oder mehreren $C_{1-4}$-Alkylgruppen substituiert ist.

**12.** Zusammensetzung nach Anspruch 9, dadurch gekennzeichnet, daß das 1,3,5-Triazinderivat unter den Verbindungen der Formel (I) ausgewählt ist, die alle folgenden Eigenschaften aufweisen:

- $X_2$ ist Sauerstoff;
- $X_3$ bedeutet die Gruppe -NH-;
- $R_3$ ist ausgewählt unter: einer geradkettigen oder verzweigten $C_{1-18}$-Alkylgruppe oder einer $C_{5-12}$-Cycloalkylgruppe die gegebenenfalls mit einer oder mehreren $C_1$-4-Alkylgruppen substituiert ist;
- $R_1$ ist ausgewählt unter: Wasserstoff; einem Alkalimetall; einer Ammoniumgruppe; einer Gruppe der Formel (IV); einer geradkettigen oder verzweigten $C_{1-18}$-Alkylgruppe; einer $C_{5-12}$-Cycloalkylgruppe, die gegebenenfalls mit einer oder mehreren $C_{1-4}$-Alkylgruppen substituiert ist;
- $R_2$ ist ausgewählt unter: Wasserstoff; einem Alkalimetall; einer Ammoniumgruppe; einer Gruppe der Formel (IV); einer geradkettigen oder verzweigten $C_{1-18}$-Alkylgruppe; einer $C_{5-12}$-Cycloalkylgruppe, die gegebenenfalls mit einer oder mehreren $C_{1-4}$-Alkylgruppen substituiert ist.

**13.** Zusammensetzung nach Anspruch 9, dadurch gekennzeichnet, daß das 1,3,5-Triazinderivat der folgenden Formel entspricht:

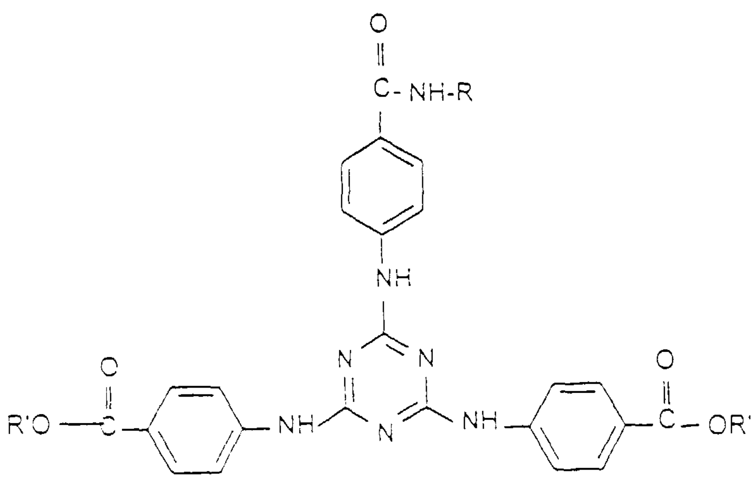

worin R' 2-Ethylhexyl und R *tert.*-Butyl bedeutet.

**14.** Zusammensetzung nach Anspruch 9, dadurch gekennzeichnet, daß das 1,3,5-Triazinderivat unter den Verbindungen der Formel (I) ausgewählt ist, die alle folgenden Eigenschaften aufweisen:

- $X_2$ und $X_3$ sind identisch und bedeuten Sauerstoff;
- $R_1$, $R_2$ und $R_3$ sind identisch und bedeuten eine $C_{6-12}$-Alkylgruppe oder eine polyethoxylierte Gruppe mit 1 bis 6 Ethylenoxideinheiten, deren OH-Endgruppe methyliert ist.

15. Zusammensetzung nach Anspruch 9, dadurch gekennzeichnet, daß das 1,3,5-Triazinderivat der folgenden Formel entspricht:

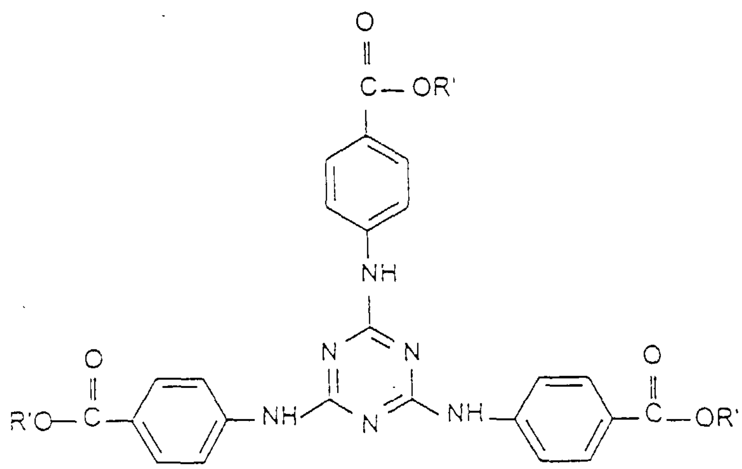

worin R' 2-Ethylhexyl bedeutet.

16. Zusammensetzung nach einem der Ansprüche 9 bis 15, dadurch gekennzeichnet, daß das 1,3,5-Triazinderivat in der Zusammensetzung in einem Mengenanteil von 0,5 bis 20 Gew.-% und vorzugsweise von 1 bis 10 Gew.-%, bezogen auf das Gesamtgewicht der Zusammensetzung, vorliegt.

17. Zusammensetzung nach einem der vorhergehenden Ansprüche, dadurch gekennzeichnet, daß das Polyaminpolymer in neutralisierter Form eingearbeitet wird.

18. Kosmetische Zusammensetzung nach einem der vorhergehenden Ansprüche, dadurch gekennzeichnet, daß sie ferner mindestens ein Nanopigment enthält.

19. Kosmetische Zusammensetzung nach dem vorhergehenden Anspruch, dadurch gekennzeichnet, daß das Nanopigment ein Titanoxid-Nanopigment ist.

20. Kosmetische Zusammensetzung nach einem der vorhergehenden Ansprüche 18 oder 19, dadurch gekennzeichnet, daß die Konzentration der Metalloxid-Nanopigmente in der Zusammensetzung im Bereich von 0,1 bis 20 Gew.-% und vorzugsweise von 0,25 bis 15 Gew.-%, bezogen auf das Gesamtgewicht der Zusammensetzung, liegt.

21. Zusammensetzung nach einem der vorhergehenden Ansprüche, dadurch gekennzeichnet, daß sie in Form einer Öl-in-Wasser-Emulsion vorliegt.

22. Verwendung eines Polyaminpolymers nach einem der Ansprüche 1 bis 4 als Antioxidationsmittel für Dibenzoylmethanderivate.

23. Verwendung eines Polyaminpolymers nach einem der Ansprüche 1 bis 4 in kosmetischen und/oder dermatologischen Zusammensetzungen, die ein Dibenzoylmethanderivat nach einem der Ansprüche 1, 6 und 7 enthalten, oder zur Herstellung dieser Zusammensetzungen zur Verbesserung der Stabilität des Dibenzoylmethanderivats gegenüber UV-Strahlung (Photostabilität) in diesen Zusammensetzungen.

24. Verfahren zur Verbesserung der Stabilität von kosmetischen und/oder dermatologischen Zusammensetzungen, die ein Dibenzoylmethanderivat nach einem der Ansprüche 1, 6 und 7 enthalten, gegenüber UV-Strahlung, dadurch gekennzeichnet, daß in die Zusammensetzung eine wirksame Menge eines Polyaminpolymers nach einem der Ansprüche 1 bis 4 eingearbeitet wird.

25. Verwendung eines Polyaminpolymers nach einem der Ansprüche 1 bis 4 in kosmetischen und/oder dermatologischen Zusammensetzungen, die ein Dibenzoylmethanderivat und ein 1,3,5-Triazinderivat nach einem der Ansprüche 9 bis 15 in Kombination enthalten, zur Verbesserung der Stabilität des 1,3,5-Triazinderivats gegenüber UV-Strahlung in diesen Zusammensetzungen oder seine Verwendung zur Herstellung dieser Zusammensetzungen.

**26.** Verfahren zur Verbesserung der Stabilität von kosmetischen und/oder dermatologischen Zusammensetzungen, die ein Dibenzoylmethanderivat und ein 1,3,5-Triazinderivat nach einem der Ansprüche 9 bis 15 in Kombination enthalten, das darin besteht, in die Zusammensetzung eine wirksame Menge eines Polyaminpolymers nach einem der Ansprüche 1 bis 4 einzuarbeiten.

## Claims

**1.** Cosmetic and/or dermatological composition comprising, in a cosmetically and/or dermatologically acceptable vehicle:

a) at least one polyamino polymer chosen from:

(A) a polyalkylene polyamine or a polyalkylene polyamine derivative chosen from:

(i) polyalkylene polyamines comprising from 7 to 20,000 repeat units;
(ii) alkylated derivatives of polyalkylene polyamines;
(iii) addition products of alkylcarboxylic acids with polyalkylene polyamines;
(iv) addition products of ketones and aldehydes with polyalkylene polyamines;
(v) addition products of isocyanates and isothiocyanates with polyalkylene polyamines;
(vi) addition products of alkylene oxide or of poly(alkylene oxide) block polymers with polyalkylene polyamines;
(vii) quaternized derivatives of polyalkylene polyamines;
(viii) addition products of a silicone with polyalkylene polyamines;
(ix) a copolymer of dicarboxylic acid and of polyalkylene polyamines;

(B) polyvinylimidazoles;
(C) polyvinylpyridines;
(D) addition products of 1-vinylimidazole monomers of formula (I):

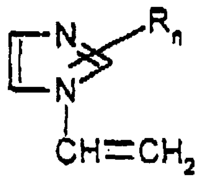

(I)

in which the R radicals, which are identical or different, represent H or a saturated or unsaturated, linear or cyclic, $C_1$-$C_6$ alkyl radical,
n is an integer ranging from 1 to 3,
with polyalkylene polyamines (A)(i) to (A)(ix);
(E) polymers based on amino acids containing a basic side chain;
(F) crosslinked derivatives of the polymers (A)(i) to (A)(ix), (B), (C), (D) and (E);

b) at least one dibenzoylmethane derivative corresponding to the following formula (V):

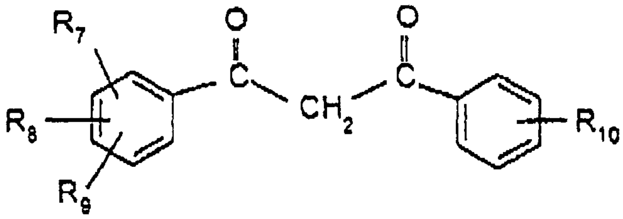

(V)

in which $R_7$, $R_8$, $R_9$ and $R_{10}$, which are identical or different, independently represent hydrogen or a hydroxyl radical or a linear or branched $C_1$-$C_8$ alkyl radical or a linear or branched $C_1$-$C_8$ alkoxy radical.

**2.** Composition according to the preceding claim, characterized in that the polyamino polymer is chosen from those comprising at least 5% of tertiary amine functional groups, advantageously at least 10% of tertiary amine functional groups and more preferably still at least 20%.

**3.** Composition according to either one of the preceding claims, characterized in that the polyamino polymer is chosen from:

(A)

(i) hyperbranched polyethyleneimines;
(ii) alkylated polyethyleneimine derivatives;
(iii) addition products of alkylcarboxylic acids with polyethyleneimine;
(iv) addition products of ketones and aldehydes with polyethyleneimine;
(v) addition products of isocyanates and isothiocyanates with polyethyleneimine;
(vi) addition products of alkylene oxide or of poly(alkylene oxide) block polymers with polyethyleneimine;
(vii) quaternized derivatives of polyethyleneimine;
(viii) addition products of a silicone with polyethyleneimine;
(ix) a copolymer of dicarboxylic acid and of polyethyleneimine;

(B) polyvinylimidazoles.

**4.** Composition according to any one of the preceding claims, characterized in that the polyamino polymer is chosen from hyperbranched polyethyleneimines.

**5.** Composition according to any one of Claims 1 to 4, characterized in that the polyamino polymer is present in the composition at a content ranging from 0.5% to 20% by weight with respect to the total weight of the composition, preferably from 1% to 10% by weight with respect to the total weight of the composition.

**6.** Composition according to any one of the preceding claims, characterized in that the dibenzoylmethane derivative is chosen from;

- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoyl methane,
- 2,6-dimethyl-4-tert-butyl-4'-methoxy dibenzoylmethane,
- 4,4'-dimethoxydibenzoylmethane.

**7.** Composition according to any one of the preceding claims, characterized in that the dibenzoylmethane derivative is 4-tert-butyl-4'-methoxydibenzoylmethane.

**8.** Composition according to any one of the preceding claims, characterized in that the dibenzoylmethane derivative is present in the composition at a content ranging from 0.2% to 15% by weight with respect to the total weight of the composition, preferably from 0.2% to 10% by weight with respect to the total weight of the composition.

**9.** Cosmetic composition according to any one of the preceding claims, characterized in that it additionally comprises: at least one 1,3,5-triazine derivative corresponding to the following formula (I):

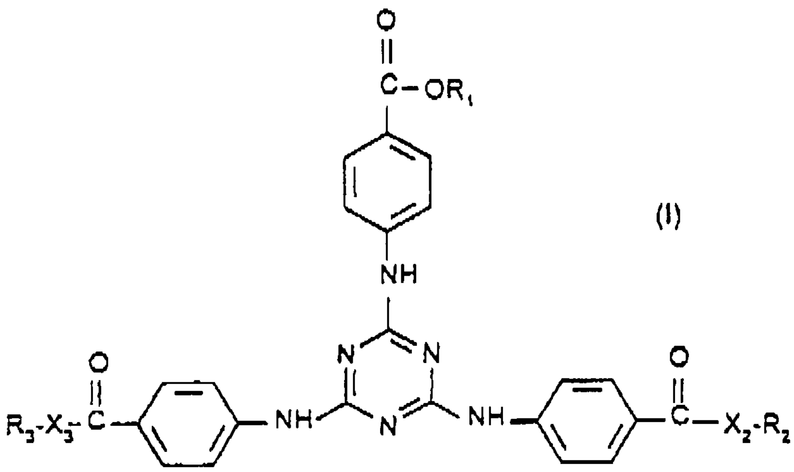

(I)

in which:

- $X_2$ and $X_3$, which are identical or different, represent oxygen or the -NH- radical;
- $R_1$, $R_2$ and $R_3$, which are identical or different, are chosen from: hydrogen; an alkali metal; an ammonium radical optionally substituted by one or more alkyl or hydroxyalkyl radicals; a linear or branched $C_1$-$C_{18}$ alkyl radical; a $C_5$-$C_{12}$ cycloalkyl radical optionally substituted by one or more $C_1$-$C_4$ alkyl radicals; a polyoxyethylene radical comprising from 1 to 6 ethylene oxide units, the terminal OH group of which is methylated; a radical of following formula (II), (III) or (IV):

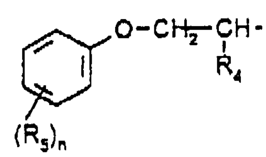

(II)

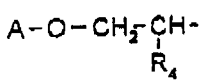

(III)

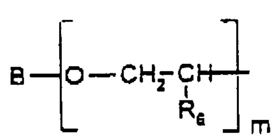

(IV)

in which:

- $R_4$ is hydrogen or a methyl radical;
- $R_5$ is a $C_1$-$C_9$ alkyl radical;
- n is an integer ranging from 0 to 3;
- m is an integer ranging from 1 to 10;
- A is a $C_4$-$C_8$ alkyl radical or a $C_5$-$C_8$ cycloalkyl radical;
- B is chosen from: a linear or branched $C_1$-$C_8$ alkyl radical; a $C_5$-$C_8$ cycloalkyl radical; an aryl radical optionally substituted by one or more $C_1$-$C_4$ alkyl radicals;
- $R_6$ is hydrogen or a methyl radical.

**10.** Composition according to the preceding claim, characterized in that the 1,3,5-triazine derivative is chosen from those of formula (I) exhibiting all the following characteristics:

- $X_2$ and $X_3$ are identical and represent oxygen;
- $R_1$ is chosen from: a $C_5$-$C_{12}$ cycloalkyl radical optionally substituted by one or more $C_1$-$C_4$ alkyl radicals; a radical of formula (II), (III) or (IV) in which:
- B is a $C_1$-$C_4$ alkyl radical;
- $R_6$ is the methyl radical;
- $R_2$ and $R_3$, which are identical or different, are chosen from: hydrogen; an alkali metal; an ammonium radical optionally substituted by one or more alkyl or hydroxyalkyl radicals; a linear or branched $C_1$-$C_{18}$ alkyl radical; a $C_5$-$C_{12}$ cycloalkyl radical optionally substituted by one or more $C_1$-$C_4$ alkyl radicals; a radical of formula (II), (III) or (IV) in which:
- B is a $C_1$-$C_4$ alkyl radical;
- $R_6$ is the methyl radical.

**11.** Composition according to Claim 9, characterized in that the 1,3,5-triazine derivative is chosen from those of formula (I) exhibiting all the following characteristics:

- $X_2$ and $X_3$ are identical and represent the -NH- radical;
- $R_3$ is chosen from: a linear or branched $C_1$-$C_{18}$ alkyl radical; a $C_5$-$C_{12}$ cycloalkyl radical optionally substituted by one or more $C_1$-$C_4$ alkyl radicals;
- $R_1$ is chosen from: hydrogen; an alkali metal; an ammonium radical; a radical of formula (IV); a linear or branched $C_1$-$C_{18}$ alkyl radical; a $C_5$-$C_{12}$ cycloalkyl radical optionally substituted by one or more $C_1$-$C_4$ alkyl radicals;
- $R_2$ is chosen from: a linear or branched $C_1$-$C_{18}$ alkyl radical; a $C_5$-$C_{12}$ cycloalkyl radical optionally substituted by one or more $C_1$-$C_4$ alkyl radicals.

**12.** Composition according to Claim 9, characterized in that the 1,3,5-triazine derivative is chosen from those exhibiting all the following characteristics:

- $X_2$ is oxygen;
- $X_3$ is the -NH- radical;
- $R_3$ is chosen from: a linear or branched $C_1$-$C_{18}$ alkyl radical; a $C_5$-$C_{12}$ cycloalkyl radical optionally substituted by one or more $C_1$-$C_4$ alkyl radicals;
- $R_1$ is chosen from: hydrogen; an alkali metal; an ammonium radical; a radical of formula (IV); a linear or branched $C_1$-$C_{18}$ alkyl radical; a $C_5$-$C_{12}$ cycloalkyl radical optionally substituted by one or more $C_1$-$C_4$ alkyl radicals;
- $R_2$ is chosen from hydrogen; an alkali metal; an ammonium radical; a radical of formula (IV); a linear or branched $C_1$-$C_{18}$ alkyl radical; a $C_5$-$C_{12}$ cycloalkyl radical optionally substituted by one or more $C_1$-$C_4$ alkyl radicals.

**13.** Composition according to Claim 9, characterized in that the 1,3,5-triazine derivative is that corresponding to the following formula:

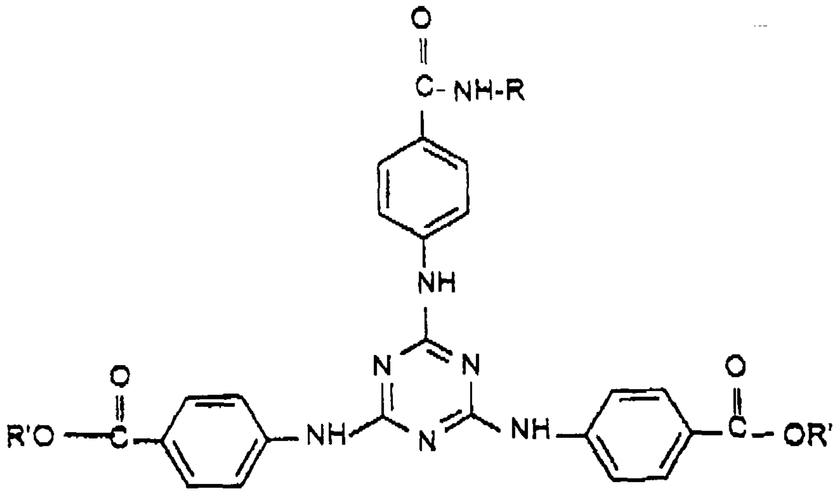

in which R' denotes a 2-ethylhexyl radical and R denotes a tert-butyl radical.

**14.** Composition according to Claim 9,
characterized in that the 1,3,5-triazine derivative is chosen from those exhibiting all the following characteristics:

- $X_2$ and $X_3$ are identical and represent oxygen;
- $R_1$, $R_2$ and $R_3$ are identical and represent a $C_6$-$C_{12}$ alkyl radical or a polyoxyethylene radical comprising from 1 to 6 ethylene oxide units, the terminal OH group of which is methylated.

**15.** Composition according to Claim 9, characterized in that the 1,3,5-triazine derivative is that corresponding to the following formula:

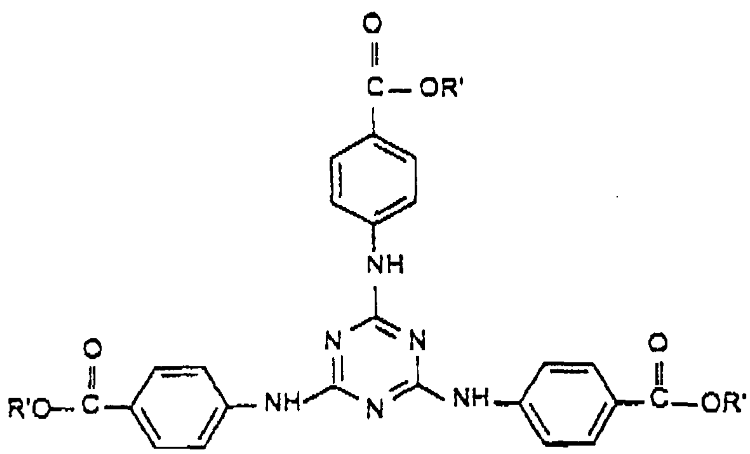

in which R' denotes a 2-ethylhexyl radical.

**16.** Composition according to any one of Claims 9 to 15, characterized in that the 1,3,5-triazine derivative is present in the composition at a content ranging from 0.5% to 20% by weight with respect to the total weight of the composition, preferably from 1% to 10% by weight with respect to the total weight of the composition.

**17.** Composition according to any one of the preceding claims, characterized in that the polyamino polymer is introduced in a neutralized form.

**18.** Cosmetic composition according to any one of the preceding claims, characterized in that it additionally comprises

at least one nanopigment.

**19.** Cosmetic composition according to the preceding claim, characterized in that the nanopigment is a titanium oxide nanopigment.

**20.** Cosmetic composition according to either one of the preceding Claims 18 and 19, characterized in that the concentration of metal oxide nanopigments in the composition is between 0.1 and 20% by weight with respect to the total weight of the composition and preferably between 0.25 and 15%.

**21.** Composition according to any one of the preceding claims, characterized in that it is in the form of an oil-in-water emulsion.

**22.** Use of a polyamino polymer as defined in any one of Claims 1 to 4, as antioxidizing agent with respect to dibenzoylmethane derivatives.

**23.** Use of a polyamino polymer as defined in any one of Claims 1 to 4 in, or for the manufacture of, cosmetic and/or dermatological compositions containing a dibenzoylmethane derivative as defined in any one of Claims 1, 6 and 7, for the purpose of improving, in the said compositions, the stability to UV radiation of the said dibenzoylmethane derivative.

**24.** Process for improving the stability to UV radiation of cosmetic and/or dermatological compositions comprising a dibenzoylmethane derivative as defined in any one of Claims 1, 6 and 7, characterized in that it consists in introducing, into the said compositions, an effective amount of a polyamino polymer as defined in any one of Claims 1 to 4.

**25.** Use of a polyamino polymer as defined in any one of Claims 1 to 4 in, or for the manufacture of, cosmetic and/or dermatological compositions containing a dibenzoylmethane derivative in combination with at least one 1,3,5-triazine derivative as defined in any one of Claims 9 to 15, for the purpose of improving, in the said compositions, the stability to UV radiation of the said 1,3,5-triazine derivative.

**26.** Process for improving the stability to UV radiation of a cosmetic and/or dermatological composition comprising a dibenzoylmethane derivative and a 1,3,5-triazine derivative as defined in any one of Claims 9 to 15, the said process consisting in introducing, into the said composition, an effective amount of a polyamino polymer as defined in any one of Claims 1 to 4.